(19) Europäisches Patentamt
European Patent Office
Office européen des brevets

(11) **EP 4 579 593 B1**

(12) **EUROPEAN PATENT SPECIFICATION**

(45) Date of publication and mention
of the grant of the patent:
**20.05.2026 Bulletin 2026/21**

(21) Application number: **24223308.8**

(22) Date of filing: **26.12.2024**

(51) International Patent Classification (IPC):
***G06T 7/11*** *(2017.01)*     ***G06T 7/174*** *(2017.01)*

(52) Cooperative Patent Classification (CPC):
**G06T 7/11; G06T 7/174;** G06T 2207/10088;
G06T 2207/20081; G06T 2207/20084;
G06T 2207/30101; G06T 2207/30172

(54) **SYSTEMS AND METHODS FOR IMAGE PROCESSING**

SYSTEME UND VERFAHREN ZUR BILDVERARBEITUNG

SYSTÈMES ET PROCÉDÉS DE TRAITEMENT D'IMAGE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **26.12.2023 CN 202311813012
27.12.2023 CN 202311828189
27.12.2023 CN 202311826362**

(43) Date of publication of application:
**02.07.2025 Bulletin 2025/27**

(73) Proprietor: **Shanghai United Imaging Healthcare
Co., Ltd.
Shanghai 201807 (CN)**

(72) Inventors:
• **YANG, Xiong**
**Shanghai, 201807 (CN)**
• **MAO, Yufei**
**Shanghai, 201807 (CN)**
• **DONG, Jinhua**
**Shanghai, 201807 (CN)**
• **SUN, Wanqing**
**Shanghai, 201807 (CN)**
• **LIU, Xuyang**
**Shanghai, 201807 (CN)**

(74) Representative: **Wang, Bo**
**Panovision IP**
**Ebersberger Straße 3**
**85570 Markt Schwaben (DE)**

(56) References cited:
**US-A1- 2023 104 945**     **US-A1- 2023 206 444**
**US-A1- 2023 237 648**

• **AL-FAHSI RESHA DWIKA HEFNI ET AL:
"MSGNet: Modified MobileNet-ShuffleNet-
GhostNet Network for Lightweight Retinal Vessel
Segmentation", 2023 10TH INTERNATIONAL
CONFERENCE ON INFORMATION
TECHNOLOGY, COMPUTER, AND ELECTRICAL
ENGINEERING (ICITACEE), IEEE, 31 August 2023
(2023-08-31), pages 94 - 99, XP034445109, DOI:
10.1109/ICITACEE58587.2023.10276687**

## Description

### CROSS-REFERENCE TO RELATED APPLICATIONS

This application claims priority to Chinese Patent Application No. 202311828189.9, filed on December 27, 2023, Chinese Patent Application No. 202311813012.1, filed on December 26, 2023, and Chinese Patent Application No. 202311826362.1, filed on December 27, 2023.

### TECHNICAL FIELD

The present disclosure generally relates to imaging processing, and more particularly, relates to systems and methods for processing images generated using multiple imaging modalities.

### BACKGROUND

In recent years, the incidence and mortality rates of cerebrovascular diseases have been steadily increasing globally, making them one of the leading causes of death. Medical imaging techniques play a crucial role in diagnosing and treating these conditions. Therefore, it is desirable to provide advanced systems and methods that can rapidly and accurately process medical images, which can improve the efficiency and accuracy of the diagnosis and/or treatment of the cerebrovascular diseases.

Systems and methods for automated assessment of a vessel including segmentation of reference walls and lumen of the vessel from one or more input medical images are described in patent application US 2023/237648 A1, published on July 27, 2023.

Methods and systems aligning and segmenting a plurality of image sequences relating to a blood vessel, including image sequences acquired using a black blood imaging sequence, are presented in patent application US 2023/206444 A1, published 29 June, 2023.

SGNet (Modified MobileNet-ShuffleNet-GhostNet Network), a lightweight network for retinal vessel segmentation leveraging components from lightweight architectures, i.e., depthwise separable convolution, channel shuffle operation, and a Ghost module, is proposed in the study of Resha Dwika Hefni Al-Fahsi et al.: "MSGNet: Modified MobileNet-ShuffleNet-GhostNet Network for Lightweight Retinal Vessel Segmentation", 2023 10th International Conference on Information Technology, Computer, and Electrical Engineering (ICITACEE), published 31 August 2023.

### SUMMARY

According to an aspect of the present disclosure, a method for image processing is provided. The method may be implemented on a computing device having at least one processor and at least one storage device. The method may include obtaining multiple black blood images of a first region of a target subject. The methods may further include generating a first segment result by inputting the black blood images into multiple input channels of a blood vessel segmentation model. The first segment result may include blood vessel segments of first blood vessels in the first region, the blood vessel segmentation model may be a trained machine learning model, and each of the black blood images may be randomly designated as an input image of one of the input channels.

According to another aspect of the present disclosure, a method for image processing is provided. The method may be implemented on a computing device having at least one processor and at least one storage device. The method may include obtaining a first image of a target subject. The first image may indicate the vascular wall in a first region of the target subject. The method may include obtaining a second image of the target subject. The second image may indicate the vascular lumen in a second region of the target subject, and the first region and the second region may both include a target region of the target subject. The method may also include generating a registration result by registering the first image and the second image, and generating a target image based on the registration result, the first image, and the second image.

According to still another aspect of the present disclosure, a method for image processing is provided. The method may be implemented on a computing device having at least one processor and at least one storage device. The method may include obtaining a first image of a first region of a target subject. The method may include obtaining a third image of a third region of the target subject. Both the first region and the third region may include a target region. The method may also include determining first detection information of the target region by processing the first image, and determining second detection information of the target region by processing the third image. The method may further include determining at least one abnormal vascular region in the target region based on the first detection information and the second detection information.

According to yet another aspect of the present disclosure, a system for image processing is provided. The system may include a storage device and a processor connected to the storage device. The storage device may be configured to store a computer instruction. When executing the computer instruction, the processor may cause the system to perform the following operations. The operations may include obtaining multiple black blood images of a first region of a target subject. The operations may further include generating a first segment result by inputting the black blood images into multiple input channels of a blood vessel segmentation model. The first segment result may include blood vessel segments of first blood vessels in the first region, the blood vessel segmentation model may be a trained machine learning model, and each of the black blood images may be randomly designated as an input image of one of the input channels.

Additional features will be set forth in part in the description which follows, and in part will become apparent to those skilled in the art upon examination of the following and the accompanying drawings or may be learned by production or operation of the examples. The features of the present disclosure may be realized and attained by practice or use of various aspects of the methodologies, instrumentalities, and combinations set forth in the detailed examples discussed below.

**BRIEF DESCRIPTION OF THE DRAWINGS**

The present disclosure is further described in terms of exemplary embodiments. These exemplary embodiments are described in detail with reference to the drawings. These embodiments are non-limiting exemplary embodiments, in which like reference numerals represent similar structures throughout the several views of the drawings, and wherein:

FIG. 1 is a schematic diagram illustrating an exemplary imaging system according to some embodiments of the present disclosure;

FIG. 2 is a block diagram illustrating an exemplary processing device according to some embodiments of the present disclosure;

FIG. 3 is a schematic diagram illustrating an exemplary process for image processing according to some embodiments of the present disclosure;

FIG. 4 is a schematic diagram illustrating an exemplary process for generating a centerline of first blood vessels according to some embodiments of the present disclosure;

FIG. 5 is a schematic diagram illustrating an exemplary blood vessel segmentation model according to some embodiments of the present disclosure;

FIG. 6 is a flowchart illustrating an exemplary process for generating a blood vessel segmentation model according to some embodiments of the present disclosure;

FIG. 7 is a schematic diagram illustrating an exemplary process for determining a centerline of first blood vessels according to some embodiments of the present disclosure;

FIG. 8 is a schematic diagram illustrating an exemplary process for determining a centerline of first blood vessels according to some embodiments of the present disclosure;

FIG. 9A is a flowchart illustrating an exemplary process for generating a target image according to some embodiments of the present disclosure;

FIG. 9B is a flowchart illustrating an exemplary process for generating a target image according to some embodiments of the present disclosure;

FIG. 10 is a schematic diagram illustrating an exemplary target image according to some embodiments of the present disclosure;

FIG. 11 is a schematic diagram illustrating an exemplary presentation interface according to some embodiments of the present disclosure;

FIG. 12 is a schematic diagram illustrating an exemplary target image according to some embodiments of the present disclosure;

FIG. 13 is a schematic diagram illustrating an exemplary plaque determination model according to some embodiments of the present disclosure;

FIG. 14 is a flowchart illustrating an exemplary process for determining at least one abnormal vascular region according to some embodiments of the present disclosure;

FIG. 15A is a schematic diagram illustrating an exemplary presentation interface according to some embodiments of the present disclosure;

FIG. 15B is a schematic diagram illustrating an exemplary presentation interface according to some embodiments of the present disclosure; and

FIG. 16 is a schematic diagram illustrating an exemplary imaging device according to some embodiments of the present disclosure.

## DETAILED DESCRIPTION

In the following detailed description, numerous specific details are set forth by way of examples in order to provide a thorough understanding of the relevant disclosure. However, it should be apparent to those skilled in the art that the present disclosure may be practiced without such details. In other instances, well-known methods, procedures, systems, components, and/or circuitry have been described at a relatively high level, without detail, in order to avoid unnecessarily obscuring aspects of the present disclosure. Various modifications to the disclosed embodiments will be readily apparent to those skilled in the art, and the general principles defined herein may be applied to other embodiments and applications without departing from the scope of the present disclosure. Thus, the present disclosure is not limited to the embodiments shown, but to be accorded the widest scope consistent with the claims.

The terminology used herein is for the purpose of describing particular example embodiments only and is not intended to be limiting. As used herein, the singular forms "a," "an," and "the" may be intended to include the plural forms as well, unless the context clearly indicates otherwise. It will be further understood that the terms "comprise," "comprises," and/or "comprising," "include," "includes," and/or "including," when used in this specification, specify the presence of stated features, integers, steps, operations, elements, and/or components, but do not preclude the presence or addition of one or more other features, integers, steps, operations, elements, components, and/or groups thereof.

It will be understood that when a unit, engine, module, or block is referred to as being "on," "connected to," or "coupled to," another unit, engine, module, or block, it may be directly on, connected or coupled to, or communicate with the other unit, engine, module, or block, or an intervening unit, engine, module, or block may be present, unless the context clearly indicates otherwise. As used herein, the term "and/or" includes any and all combinations of one or more of the associated listed items.

These and other features, and characteristics of the present disclosure, as well as the methods of operation and functions of the related elements of structure and the combination of parts and economies of manufacture, may become more apparent upon consideration of the following description with reference to the accompanying drawings, all of which form a part of this disclosure. It is to be expressly understood, however, that the drawings are for the purpose of illustration and description only and are not intended to limit the scope of the present disclosure. It is understood that the drawings are not to scale.

Provided herein are systems and methods for non-invasive biomedical imaging/treatment, such as for disease diagnosis, disease therapy, or research purposes. In some embodiments, the systems may include an imaging system. The imaging system may include a single modality system and/or a multi-modality system. The term "modality" used herein broadly refers to an imaging or treatment method or technology that gathers, generates, processes, and/or analyzes imaging information of a subject or treatments the subject. The single modality system may include, for example, an ultrasound imaging system, an X-ray imaging system, a computed tomography (CT) system, a magnetic resonance imaging (MRI) system, an ultrasonography system, a positron emission tomography (PET) system, an optical coherence tomography (OCT) imaging system, an ultrasound (US) imaging system, an intravascular ultrasound (IVUS) imaging system, a near-infrared spectroscopy (NIRS) imaging system, a digital subtraction angiography (DSA) system, or the like, or any combination thereof. The multi-modality system may include, for example, an X-ray imaging-magnetic resonance imaging (X-ray-MRI) system, a positron emission tomography-X-ray imaging (PET-X-ray) system, a single photon emission computed tomography-magnetic resonance imaging (SPECT-MRI) system, a positron emission tomography-computed tomography (PET-CT) system, a C-arm system, a positron emission tomography-magnetic resonance imaging (PET-MR) system, a digital subtraction angiography-magnetic resonance imaging (DSA-MRI) system, etc. It should be noted that the medical system described below is merely provided for illustration purposes, and is not intended to limit the scope of the present disclosure.

In the present disclosure, the subject may include a biological object and/or a non-biological object. The biological object may be a human being, an animal, a plant, or a specific portion, organ, and/or tissue thereof. For example, the subject may include the head, the neck, the thorax, the heart, the stomach, a blood vessel, a soft tissue, a tumor, a nodule, or the like, or any combination thereof. In some embodiments, the subject may be a man-made composition of organic and/or inorganic matters that are with or without life. The terms "object" and "subject" are used interchangeably in the present disclosure.

In the present disclosure, the term "image" may refer to a two-dimensional (2D) image, a three-dimensional (3D) image, or a four-dimensional (4D) image (e.g., a time series of 3D images). In some embodiments, the term "image" may refer to an image of a region (e.g., a region of interest (ROI)) of a subject. In some embodiments, the image may be a medical image, an optical image, etc.

In the present disclosure, a representation of a subject (e.g., an object, a patient, or a portion thereof) in an image may be referred to as "subject" for brevity. For instance, a representation of an organ, tissue (e.g., a heart, a liver, a lung), or an ROI in an image may be referred to as the organ, tissue, or ROI, for brevity. Further, an image including a representation of a subject, or a portion thereof, may be referred to as an image of the subject, or a portion thereof, or an image including the subject, or a portion thereof, for brevity. Still further, an operation performed on a representation of a subject, or a portion thereof, in an image may be referred to as an operation performed on the subject, or a portion thereof, for brevity. For instance, a segmentation of a portion of an image including a representation of an ROI from the image may be referred to as a segmentation of the ROI for brevity.

The present disclosure relates to systems and methods for image processing. The methods may include obtaining multiple black blood images of a first region of a target subject. The methods may further include generating a first segment result by inputting the black blood images into multiple input channels of a blood vessel segmentation model. The first segment result may include blood vessel segments of first blood vessels in the first region, the blood vessel segmentation model may be a trained machine learning model, and each of the black blood images may be randomly designated as an input image of one of the input channels. Furthermore, a centerline of the first blood vessels may be determined based on the first segment result. By processing the black blood images using the blood vessel segmentation model, the first segment result can be determined automatically, which can reduce user intervention and improve the efficiency and accuracy of the determination of the centerline.

In addition, the methods may further include obtaining another image (e.g., a second image and/or a third image) using a different imaging modality from the black blood images, generating a registration result by registering the first image and the another image, and generating a target image based on the registration result, the first image, and the another image. By performing an analysis on the images corresponding to multiple imaging modalities, more information can be provided to users, which facilitates the user diagnosis and analysis, thereby improving the efficiency and accuracy of the vascular detection.

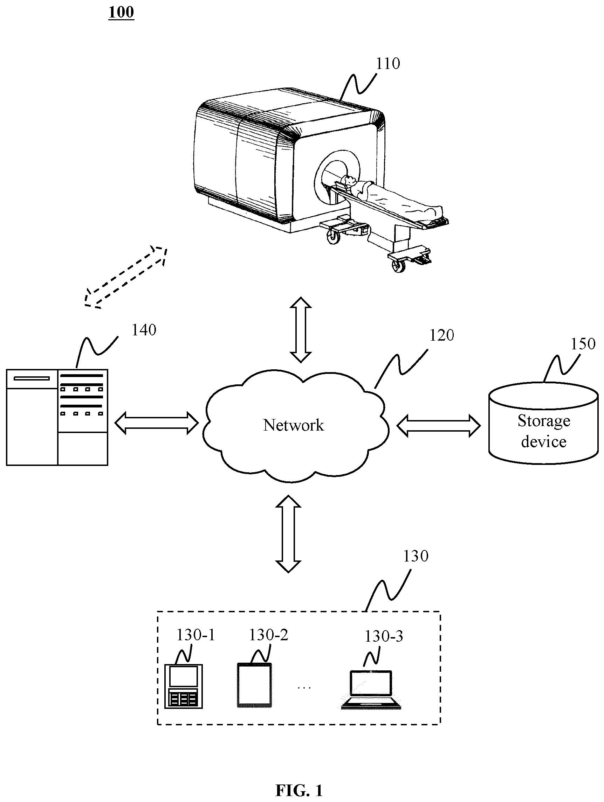

FIG. 1 is a schematic diagram illustrating an exemplary imaging system 100 according to some embodiments of the present disclosure. As shown in FIG. 1, the imaging system 100 may include an imaging device 110, a network 120, one or more terminals 130, a processing device 140, and a storage device 150.

The imaging device 110 may be configured to generate or provide image data by scanning a target subject or at least a part of the target subject. For example, the imaging device 110 may obtain multiple black blood images of a first region of the target subject by performing Magnetic Resonance Imaging (MRI) scans on the first region of the target subject. In some embodiments, the imaging device 110 may include a single modality imaging device or a multi-modality imaging device. The multi-modality scanner may perform multi-modality imaging simultaneously or in sequence. For example, the PET-MRI device may generate a structural magnetic resonance (MR) image and a functional PET image simultaneously or in sequence.

The network 120 may include any suitable network that can facilitate the exchange of information and/or data for the imaging system 100. In some embodiments, one or more components (e.g., the imaging device 110, the terminal 130, the processing device 140, the storage device 150, etc.) of the imaging system 100 may communicate information and/or data with one or more other components of the imaging system 100 via the network 120. For example, the processing device 140 may obtain image data from the imaging device 110 via the network 120. As another example, the processing device 140 may obtain user instructions from the terminal 130 via the network 120. In some embodiments, the network 120 may include one or more network access points.

The terminal(s) 130 may include a mobile device 130-1, a tablet computer 130-2, a laptop computer 130-3, or the like, or any combination thereof. In some embodiments, the mobile device 130-1 may include a smart home device, a wearable device, a mobile device, a virtual reality device, an augmented reality device, or the like, or any combination thereof. In some embodiments, the terminal(s) 130 may include a processing unit, a display unit, a sensing unit, an input/output (I/O) unit, a storage unit, etc. Exemplary display units may include a liquid crystal display (LCD), a light-emitting diode (LED)-based display, a flat panel display, a curved screen, a television device, a cathode ray tube (CRT), or the like, or any combination thereof. In some embodiments, the display unit may include an interactive interface that is configured to receive an input from a user. In some embodiments, the terminal(s) 130 may be part of the processing device 140.

The processing device 140 may process data and/or information obtained from one or more components (the imaging device 110, the terminal(s) 130, and/or the storage device 150) of the imaging system 100. For example, the processing device 140 may obtain multiple black blood images of the first region of the target subject, and generate a first segment result by inputting the black blood images into multiple input channels of a blood vessel segmentation model. As another example, the processing device 140 may determine a centerline of first blood vessels in the first region. As still another example, the processing device 140 may generate a target image. As still another example, the processing device 140 may determine at least one abnormal vascular region in a target region. In some embodiments, the processing device 140 may be a single server or a server group. The server group may be centralized or distributed. In some embodiments, the processing device 140 may be local or remote. In some embodiments, the processing device 140 may be implemented on a cloud platform.

In some embodiments, the processing device 140 may be implemented by a computing device. For example, the computing device may include a processor, a storage, an input/output (I/O), and a communication port. The processor may execute computer instructions (e.g., program codes) and perform functions of the processing device 140 in accordance with the techniques described herein. The computer instructions may include, for example, routines, programs, objects, components, data structures, procedures, modules, and functions, which perform particular functions described herein. In some embodiments, the processing device 140, or a portion of the processing device 140 may be implemented by a portion of the terminal 130.

The storage device 150 may store data/information obtained from the imaging device 110, the terminal(s) 130, and/or any other component of the imaging system 100. In some embodiments, the storage device 150 may include a mass storage, a removable storage, a volatile read-and-write memory, a read-only memory (ROM), or the like, or any combination thereof. In some embodiments, the storage device 150 may store one or more programs and/or instructions to perform exemplary methods described in the present disclosure.

In some embodiments, the imaging system 100 may include one or more additional components and/or one or more components of the imaging system 100 described above may be omitted. Additionally or alternatively, two or more components of the imaging system 100 may be integrated into a single component. A component of the imaging system 100 may be implemented on two or more sub-components.

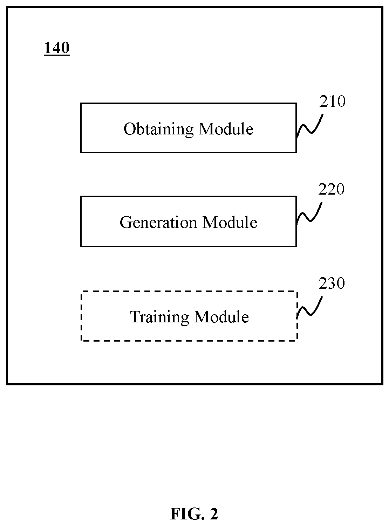

FIG. 2 is a block diagram illustrating an exemplary processing device 140 according to some embodiments of the present disclosure. The modules illustrated in FIG. 2 may be implemented on the processing device 140. In some embodiments, the processing device 140 may be in communication with a computer-readable storage medium (e.g., the storage device 150 illustrated in FIG. 1) and execute instructions stored in the computer-readable storage medium. The processing device 140 may include an obtaining module 210 and a generation module 220.

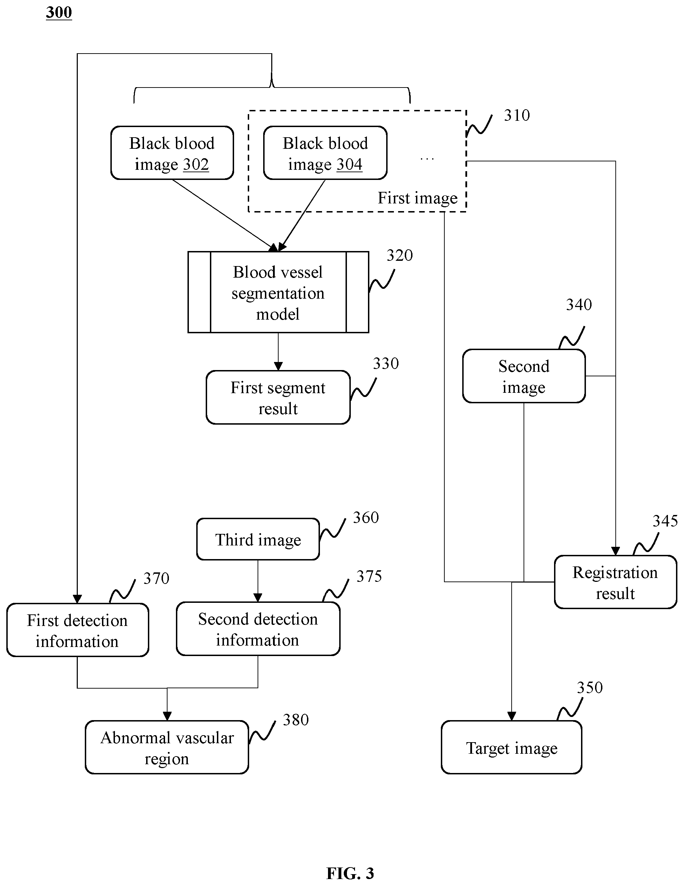

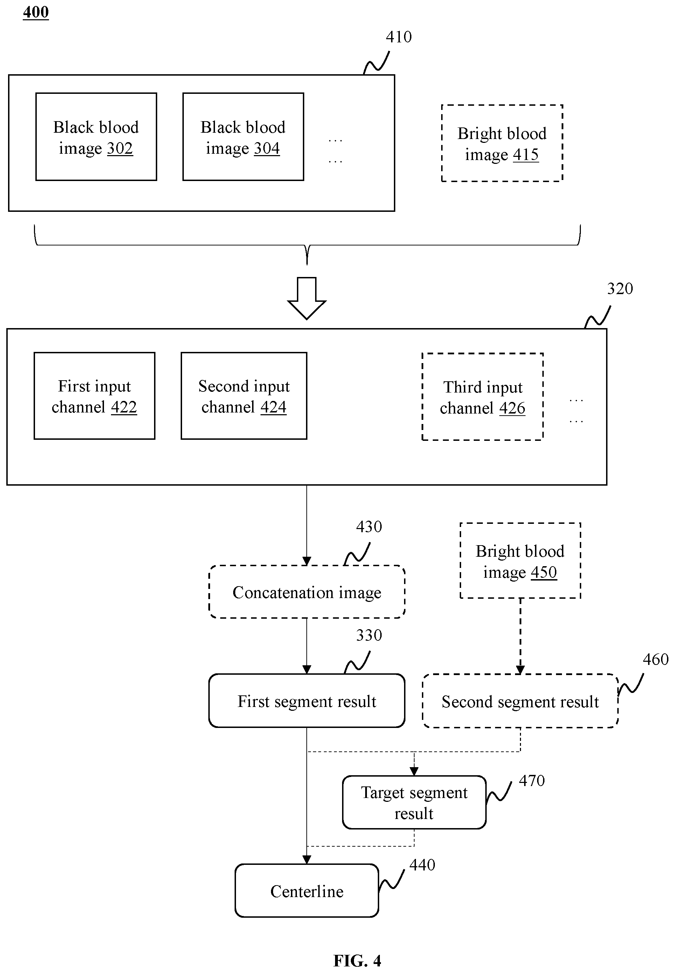

The obtaining module 210 may be configured to obtain images (e.g., multiple black blood images, a first image, a second image) of a target subject. More descriptions regarding the obtaining images may be found elsewhere in the present disclosure. See, e.g., FIGs. 3 and 4 and relevant descriptions thereof.

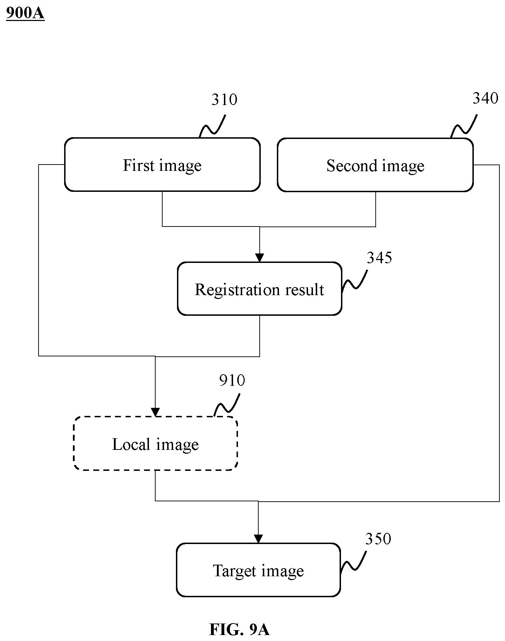

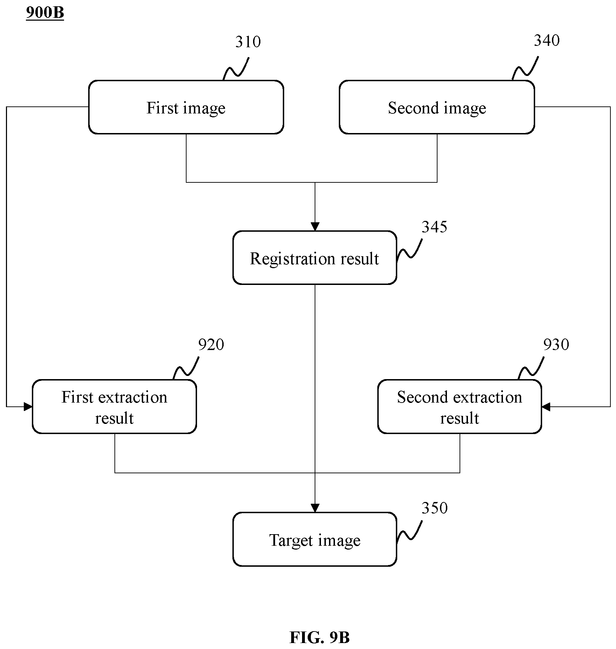

The generation module 220 may be configured to generate a first segment result by inputting the black blood images into multiple input channels of a blood vessel segmentation model. More descriptions regarding the generation of the first segment result may be found elsewhere in the present disclosure. See, e.g., FIGs. 3 and 4 and relevant descriptions thereof. The generation module 220 may be configured to generate a registration result by registering the first image and the second image, and generate a target image based on the first image, the second image, and the registration result. More descriptions regarding the generation of the first segment result may be found elsewhere in the present disclosure. See, e.g., FIGs. 9A and 9B and relevant descriptions thereof.

In some embodiments, the processing device 140 may further include a training module 230. The training module 230 may be configured to generate one or more machine learning models used for image processing, such as, the blood vessel segmentation model, an abnormal information determination model, etc. In some embodiments, the training module 230 may be implemented on the processing device 140 or a processing device other than the processing device 140. In some embodiments, the training module 230 and other modules (e.g., the obtaining module 210, the generation module 220) may be implemented on a same processing device (e.g., the processing device 140). Alternatively, the training module 230 and other modules (e.g., the obtaining module 210 and/or the generation module 220) may be implemented on different processing devices. For example, the training module 230 may be implemented on a processing device of a vendor of the machine learning model(s), while the other modules may be implemented on a processing device of a user of the machine learning model(s).

It should be noted that the above descriptions of the processing device 140 are provided for the purposes of illustration, and are not intended to limit the scope of the present disclosure. For persons having ordinary skills in the art, various variations and modifications may be conducted under the guidance of the present disclosure. However, those variations and modifications do not depart from the scope of the present disclosure. In some embodiments, the processing device 140 may include one or more other modules. For example, the processing device 140 may include a storage module to store data generated by the modules in the processing device 140. In some embodiments, any two of the modules may be combined as a single module, and any one of the modules may be divided into two or more units.

FIG. 3 is a schematic diagram illustrating an exemplary process 300 for image processing according to some embodiments of the present disclosure.

As illustrated in FIG. 3, multiple black blood images (e.g., a black blood image 302, a black blood image 304, etc.) of a first region of a target subject may be input into a blood vessel segmentation model 320, and the blood vessel segmentation model 320 may output a first segment result 330. The first segment result 330 may include blood vessel segments of first blood vessels in the first region.

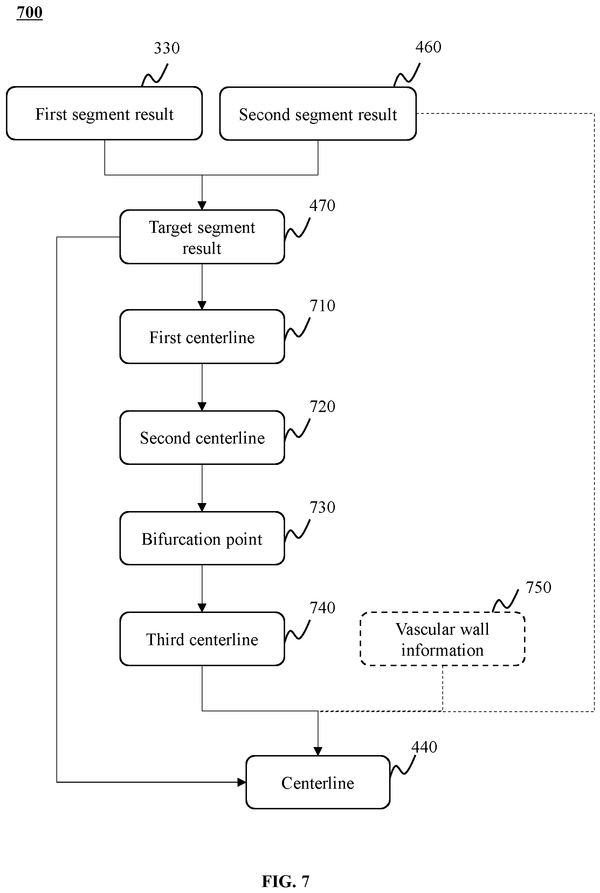

The blood vessel segmentation model 320 may be a trained machine learning model, which can determine the first segment result 330 based on the black blood images. For example, the blood vessel segmentation model 320 may include multiple input channels, and each of the black blood images may be randomly designated as an input image of one of the input channels. The blood vessel segmentation model 320 may generate a concatenation image of the input images of the multiple input channels, and generate the first segment result 330 by processing the concatenation image. More descriptions regarding the generation of the first segment result using the blood vessel segmentation model may be found elsewhere in the present disclosure. See, e.g., FIGs. 4 and 7 and relevant descriptions thereof.

In some embodiments, the black blood images may include a first image 310 (e.g., the black blood image 304, etc.). The first image 310 may indicate the vascular wall in the first region of the target subject. The processing device 140 (e.g., the obtaining module 210) may obtain a second image 340 of the target subject. The second image 340 may indicate the vascular lumen in a second region of the target subject, and the first region and the second region may both include a target region (also referred to as a region of interest (ROI)) of the target subject. Further, the processing device 140 (e.g., the generation module 220) may generate a registration result 345 by registering the first image 310 and the second image 340, and generate a target image 350 based on the registration result 345, the first image 310, and the second image 340.

Merely by way of example, the processing device 140 may determine a local image in the first image 310 corresponding to the second image 340 based on the registration result 345, and generate the target image 350 based on the local image and the second image 340. The target image 350 may include the local image and the second image 340 displayed in parallel, or the target image 350 may be a fusion image of the local image and the second image 340. As another example, the processing device 140 may determine blood vessels of interest in the first image 310 corresponding to the blood vessels of interest in the second image 340 based on the registration result 345, and generate the target image 350 that includes the first image 310 and the second image 340 displayed in parallel and includes a label corresponding to the blood vessels of interest in the first image 310. More descriptions regarding the generation of the target image may be found elsewhere in the present disclosure. See, e.g., FIGs. 9A and 9B, and relevant descriptions thereof.

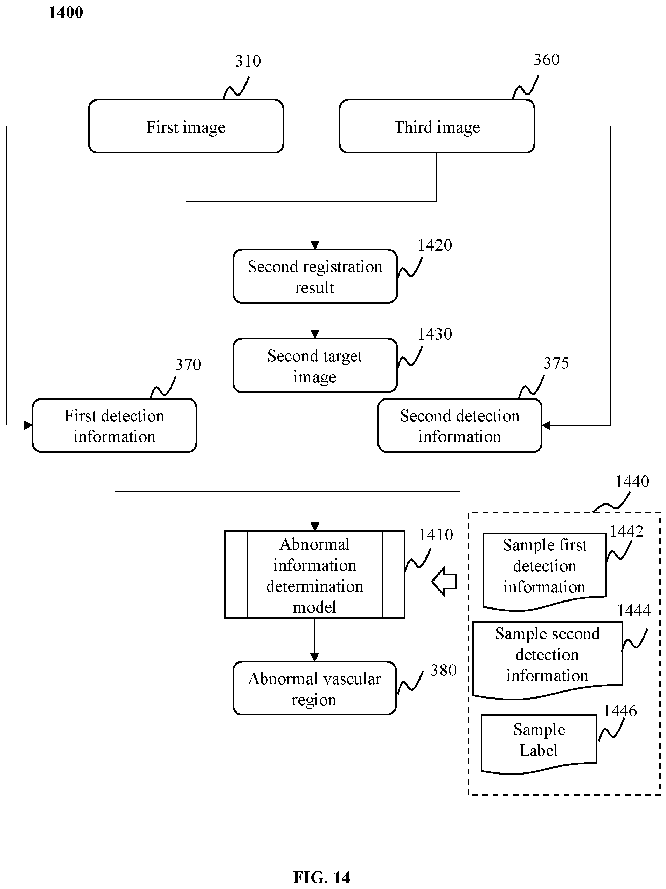

In some embodiments, the processing device 140 may further obtain a third image 360 of a third region of the target subject. The third image 360 may be acquired using a perfusion imaging sequence, and the third region 360 may include the target region. The processing device 140 may determine first detection information 370 of the target region by processing at least one of the multiple black blood images, and determine second detection information 375 of the target region by processing the third image 360. Further, the processing device 140 may determine at least one abnormal vascular region 380 in the target region based on the first detection information 370 and the second detection information 375. For example, the at least one abnormal vascular region 370 may include a plaque in the target region. More descriptions regarding the determination of the at least one abnormal vascular region may be found elsewhere in the present disclosure. See, e.g., FIG. 14 and relevant descriptions thereof.

FIG. 4 is a schematic diagram illustrating an exemplary process 400 for generating a centerline of first blood vessels according to some embodiments of the present disclosure.

The extraction of vascular centerlines is crucial for diagnosing and treating vascular diseases. Accurate centerline extraction enhances disease analysis and treatment planning. Traditionally, this process is manual or interactive, which can result in lower accuracy and efficiency. To improve both accuracy and efficiency of centerline extraction, process 400 may be performed.

As illustrated in FIG. 4, the processing device 140 (e.g., the obtaining module 210) may obtain multiple black blood images 410 (e.g., the black blood image 302, the black blood image 304, etc.) of a first region of a target subject.

The first region refers to a region of the target subject that a medical scan (e.g., an MRI scan) is performed on. For example, the first region may be a target region (also referred to as an ROI) or a region including the target region. The target region refers to a region of the target subject that needs to be treated or diagnosed. For instance, the target region may include a region that includes a lesion (e.g., a plaque, an aneurysm, a sandwich region, an ulceration, a thrombosis, an inflammation region, an obstruction, a tumor, a cancer-ridden organ, obstructed blood vessels, etc.) of the target subject.

In some embodiments, the first region may include first blood vessels. The first blood vessels may be of various types. For example, the first blood vessels may include arterial blood vessel(s), venous blood vessel(s), and/or capillary(capillaries).

A black blood image refers to an image or sequence images obtained by an imaging device (e.g., the imaging device 110) using a black blood technique.

The black blood technique is a type of MRI technique used primarily in vascular imaging to visualize structures of blood vessels. For instance, when the imaging device is used for vascular imaging, a saturation radio frequency pulse is applied before the blood flow enters an imaging volume (e.g., a lumen of the first blood vessels) to pre-saturate the blood flow. When the blood flow enters the imaging volume, another radio frequency pulse is applied, which causes that a longitudinal magnetization vector of the presaturated blood flow is relatively small, and almost no magnetic resonance (MR) signal corresponding to the blood flow is generated. Therefore, the blood flow appears as black low signals, while tissues surrounding the blood flow (also referred to as surrounding tissues) appear as high signals, thus forming the black blood image. In the black blood image, the blood within the blood vessels appears dark (black), which contrasts with the surrounding tissues that appear relatively bright.

By using the black blood technique, the contrast between the surrounding tissues (e.g., the vascular wall) and the blood can be enhanced, which allows for clearer visualization of the vascular wall and any lesions or abnormalities (e.g., plaques, clots, or other structural issues) within or adjacent to the blood vessels, thereby aiding in the diagnosis of vascular diseases and improving the accuracy of the diagnosis. For example, obstructed blood vessels in the first blood vessels can be presented in the black blood images, which can improve the subsequent determination of a centerline of the first blood vessels, thereby improving the accuracy of the diagnosis of the vascular diseases.

In some embodiments, each of the multiple black blood images 410 may be acquired using an MRI sequence.

Exemplary MRI sequences may include a T1 (longitudinal relaxation time) sequence, a contrast-enhanced T1 (T1CE) sequence, a T2 (transverse relaxation time) sequence, a fast spin echo (FSE) sequence, an inversion recovery (IR) sequence, a susceptibility-weighted imaging (SWI) sequence, a simultaneous non-contrast angiography and intraplaque hemorrhage (SNAP) sequence, a contrast-enhanced magnetic resonance angiography (ceMRA) sequence, a time of flight (TOF) sequence, or the like, or any combination thereof.

In some embodiments, the black blood image 304 may be collected using a different MRI sequence from the black blood image 302. For example, the black blood image 302 may be collected using the T1 sequence, and the black blood image 304 may be collected using the T1CE sequence. Correspondingly, the black blood image 302 and the black blood image 304 include reference information of different portions of the target subject.

In some embodiments, the black blood image 304 may be the same as the black blood image 302. For example, the black blood image 304 may be collected using a same MRI sequence as the black blood image 302. As another example, the black blood image 304 may be a copy image of the black blood image 302. The copy image refers to an image obtained by copying the black blood image 302. That is, the copy image of the black blood image 302 is the same as the black blood image 302.

In some embodiments, the processing device 140 may obtain the multiple black blood images 410 from an imaging device (e.g., the imaging device 110, an MRI device, an MRI scanner of a multi-modality imaging device, etc.) or a storage device (e.g., the storage device 150, a database, or an external storage).

In some embodiments, the processing device 140 (e.g., the generation module 220) may generate the first segment result 330 by inputting the multiple black blood images 410 into multiple input channels (e.g., a first input channel 422, a second input channel 424, etc.) of the blood vessel segmentation model 320. The first segment result 330 may include blood vessel segments of first blood vessels in the first region. For example, the processing device 140 may input the multiple black blood images 410 into the multiple input channels of the blood vessel segmentation model 320, and the blood vessel segmentation model 320 may output the first segment result 330.

In some embodiments, the blood vessel segmentation model 320 refers to a process or an algorithm for determining the first segment result 330 based on the multiple black blood images 410. For example, the blood vessel segmentation model 320 may indicate a mapping relationship between the multiple black blood images 410 of the first region and the blood vessel segments of the first blood vessels in the first region. The blood vessel segmentation model 320 may be a trained machine learning model. In some embodiments, the blood vessel segmentation model 320 may include a convolutional neural network (CNN) model, a recurrent neural network (RNN) model, a long short term memory (LSTM) network model, a fully convolutional neural network (FCN) model, a generative adversarial network (GAN) model, a radial basis function (RBF) machine learning model, a DeepMask model, a SegNet model, a dilated convolution model, a conditional random fields as recurrent neural networks (CRFasRNN) model, a pyramid scene parsing network (pspnet) model, or the like, or any combination thereof.

In some embodiments, the blood vessel segmentation model 320 may include the multiple input channels, and each of the black blood images 410 may be randomly designated as the input image of one of the input channels. Merely by way of example, the processing device 140 may designate the black blood image 302 as an input image of the first input channel 422, and designate the black blood image 304 as an input image of the second input channel 424. Alternatively, the processing device 140 may designate the black blood image 304 as an input image of the first input channel 422, and designate the black blood image 302 as an input image of the second input channel 424.

In some embodiments, to generate the first segment result 330, the blood vessel segmentation model 320 may be configured to generate a concatenation image 430 of the input images of the multiple input channels, and generate the first segment result 330 by processing the concatenation image 430. The concatenation image 430 may be a 4-dimensional image of the input images (i.e., the black blood images 410). In some embodiments, the concatenation image 430 may be represented by channel × depth × height × width (CDHW).

Merely by way of example, after randomly inputting the black blood image 302 and the black blood image 304 into the first input channel 422 and the second input channel 424, the concatenation image 430 may be generated by concatenating the black blood image 304 to the black blood image 302 or concatenating the black blood image 302 to the black blood image 304. The concatenation image 430 may be represented by Equation (1):

$$CDHW = 2 \times D_1 H_1 W_1, \qquad (1)$$

where CDHW refers to a channel count, a depth, a height, and a width of the concatenation image 430, and $D_1 H_1 W_1$ refers to a depth, a height, and a width of the black blood image 302 or the black blood image 304.

Further, the first segment result 330 may be generated by processing (e.g., performing a segmentation operation on) the concatenation image 430 using layers (e.g., lower sampling layers and upper sampling layers) of the blood vessel segmentation model 320.

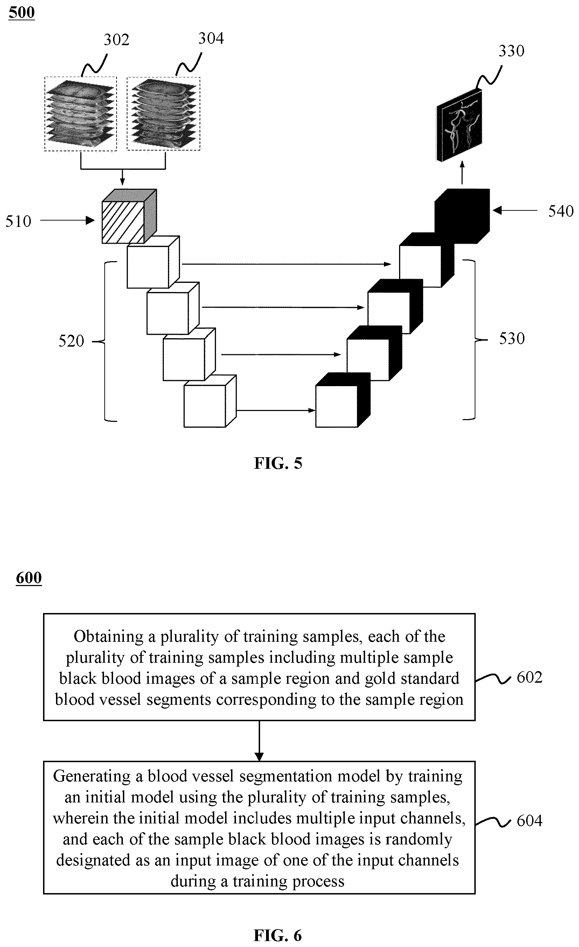

Merely by way of example, as illustrated in FIG. 5, FIG. 5 is a schematic diagram illustrating an exemplary blood vessel segmentation model 500 according to some embodiments of the present disclosure. The blood vessel segmentation model 500 may include an input layer 510 (including multiple input channels), an output layer 540, and one or more convolution layers (e.g., lower convolution layers 520, upper convolution layers 530, etc.) between the input layer 510 and the output layer 540, and each of the lower convolution layers 520 may be connected to one of the upper convolution layers 530 through a skip connection. The black blood image 302 and the black blood image 304 may be randomly input into the multiple input channels of the input layer 510 to generate a concatenation image, and segment features of each of the blood vessel segments of the first blood vessels in the first region may be obtained by performing feature extraction on the concatenation image through the lower convolution layers 520 and the upper convolution layers 530. Finally, the first segment result 330 generated by the blood vessel segmentation model 500 may be output by the output layer 540.

Since the concatenation image 430 includes the black blood image 302 and the black blood image 304, the blood vessel segmentation model 320 can effectively synthesize features of different black blood images, such that the first segment result can be accurately determined from the concatenation image.

The approach disclosed herein effectively integrates the characteristics of both types of black-blood images, allowing for accurate identification of occluded vessels. This avoids issues like poor extraction or incomplete extraction of vessel centerlines in cases of vessel blockage.

It should be noted that the blood vessel segmentation model is merely provided for illustration purposes, and can be modified according to an actual need. For example, a bright blood image of the first region of the target subject may be input into the blood vessel segmentation model with the black blood images to generate the first segment result.

Merely by way of example, as illustrated in FIG. 4, the processing device 140 may further obtain a bright blood image 415 of the first region of the target subject. The bright blood image 415 may be collected using a bright blood technique.

The bright blood technique is a type of MRI technique used to visualize a condition of the blood flow in blood vessels and the structures of the blood vessels. For instance, when the bright blood technique is used, a specific radio frequency pulse (e.g., a gradient echo T1W1 sequence) is applied. Static tissues in the imaging volume or layer are repeatedly excited and in a saturated state, and a magnetization vector of the static tissues is relatively small, thus inhibiting MR signals of the static tissues. The blood flow is not saturated by the RF pulse, and when the blood flows into the imaging volume or layer, high MR signals corresponding to the blood flows are generated, which forms a contrast with the static tissues, and generates the bright blood image.

In some embodiments, the bright blood image 415 may be collected using a second MRI sequence, and the second MRI sequence may include a phase contrast (PC) sequence, a contrast-enhanced magnetic resonance angiography (ceMRA) sequence, a time of flight (TOF) sequence, or the like, or any combination thereof.

In some embodiments, the processing device 140 may obtain the bright blood image 415 from an imaging device (e.g., the imaging device 110, an MRI device, an MRI scanner of a multi-modality imaging device, etc.) or a storage device (e.g., the storage device 150, a database, or an external storage).

The processing device 140 may generate the first segment result 330 by inputting the black blood images 410 and the bright blood image 415 into the multiple input channels of the blood vessel segmentation model 320. For example, the blood vessel segmentation model 320 may generate the concatenation image 430 of the input images (e.g., the bright blood image 415, the black blood image 302, and the black blood image 304) of the multiple input channels, and generate the first segment result 330 by processing the concatenation image 430.

Each of the black blood images 410 and the bright blood image 415 may be randomly designated as an input image of one of the input channels. For example, the processing device 140 may designate the bright blood image 415 as an input image of the first input channel 422, designate the black blood image 302 as an input image of the second input channel 424, and designate the black blood image 304 as an input image of the third input channel 426.

In some embodiments, the processing device 140 may obtain the blood vessel segmentation model from a storage device (e.g., the storage device 150) of the imaging system 100 or a third-party database. In some embodiments, the blood vessel segmentation model may be generated by the processing device 140 (e.g., the training module 230) or another computing device according to a machine learning algorithm. In some embodiments, the blood vessel segmentation model may be generated by a computing device (e.g., the processing device 140) by performing process 600 as described in connection with FIG. 6.

In some embodiments, the processing device 140 (e.g., the generation module 220) may further determine a centerline 440 of the first blood vessels in the first region based on the first segment result 330.

The centerline 440 refers to a connection line of center points in the first blood vessels. As used herein, a center point refers to a point at a center of each cross section of the first blood vessels, and a cross section refers to a section of the first blood vessels perpendicular to a blood flow in the first blood vessels. In some embodiments, the centerline 440 may be used to determine (e.g., position) the lesion in the first blood vessels (e.g., the obstructed blood vessels).

In some embodiments, the processing device 140 may determine the centerline 440 of the first blood vessels by performing a centerline extraction operation on the first segment result 330. Exemplary centerline extraction operations may include a distance transformation operation, a morphological corrosion operation, an optimum route algorithm, a model-based centerline extraction algorithm, or the like, or any combination thereof. For example, the processing device 140 may determine a sub-centerline of each blood vessel segment in the first segment result 330 by performing the centerline extraction operation on the blood vessel segment, and determine the centerline 440 by connecting the sub-centerline of each blood vessel segment. For instance, for each of the blood vessel segments in the first segment result 330, the processing device 140 may obtain the vascular wall or the vascular lumen of the blood vessel segment by performing a region segmentation on the blood vessel segment based on a grayscale of each pixel (or voxel) of the blood vessel segment, and determine the sub-centerline of the blood vessel segment by performing the distance transformation operation on the vascular wall or the vascular lumen of the blood vessel segment. As another example, the processing device 140 may divide the first segment result 330 into a first set of blood vessel segments and a second set of blood vessel segments using a non-adhesive vessel segmentation technique. The first set of blood vessel segments may include a blood vessel segment corresponding to the left external carotid artery (LECA), a blood vessel segment corresponding to the left vertebral artery (LVA), and a blood vessel segment corresponding to the right external carotid artery (RECA). The second set of blood vessel segments may include remaining blood vessel segments. The processing device 140 may determine a sub-centerline of each blood vessel segment in first set of blood vessel segments and the second set of blood vessel segments, respectively, by determining a distance field of each blood vessel segment. Finally, the processing device 140 may determine the centerline 440 by combining the sub-centerline of each blood vessel segment.

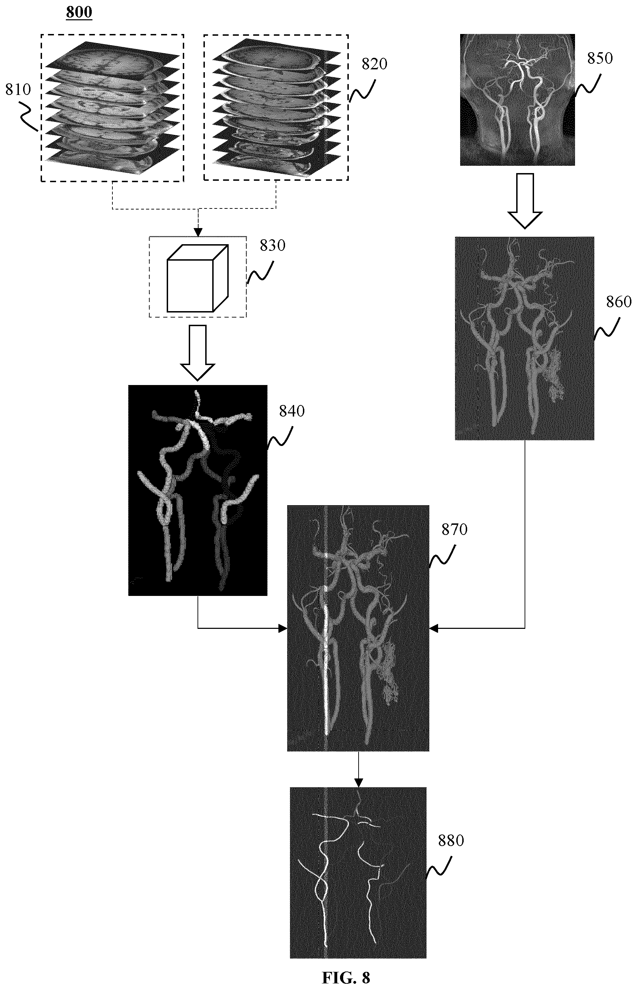

In some embodiments, the processing device 140 may further obtain a bright blood image 450 of the first region of the target subject. The bright blood image 450 may be the same as or similar to the bright blood image 415, which is not repeated herein. The processing device 140 may obtain a second segment result 460 corresponding to the bright blood image 450 by performing a segment operation on the bright blood image 450, and obtain a target segment result 470 by fusing the first segment result 330 and the second segment result 460. The second segment result 460 may include second blood vessel segments of the first blood vessels in the first region. Finally, the processing device 140 may determine the centerline 440 of the first blood vessels based on the target segment result 470. More descriptions regarding the generation of the centerline of the first blood vessels may be found elsewhere in the present disclosure. See, e.g., FIGs. 7 and 8, and relevant descriptions thereof.

In some embodiments, the processing device 140 may obtain multiple black blood images 410 (e.g., the black blood image 302 and the black blood image 304) of the first region of the target subject, and generate the first segment result 330 by inputting the black blood images 410 into multiple input channels (e.g., the first input channel 422 and the second input channel 424) of the blood vessel segmentation model 320. The first segment result 330 may include the blood vessel segments of the first blood vessels in the first region, the blood vessel segmentation model 320 may be a trained machine learning model, and each of the black blood images 410 may be randomly designated as an input image of one of the input channels.

According to some embodiments of the present disclosure, by randomly inputting the black blood images into the multiple input channels of the blood vessel segmentation model, the concatenation image can be generated in a disordered concatenation manner, which can avoid errors in the concatenation or registration of the black blood images, thereby improving the accuracy of the generation of the concatenation image and the subsequent determination of vascular centerlines. Furthermore, by generating the first segment result based on the concatenation image, the features of the black blood images can be effectively synthesized to accurately determine the first segment result from the concatenation image, thereby improving the accuracy of the determination of the first segment result. In addition, by using the blood vessel segmentation model, the first segment result and the centerline can be generated automatically, which can reduce manual intervention, thereby improving the efficiency and accuracy of the generation of the first segment result and the centerline.

FIG. 6 is a flowchart illustrating an exemplary process 600 for generating a blood vessel segmentation model according to some embodiments of the present disclosure.

In 602, the processing device 140 (e.g., the training module 230) may obtain a plurality of training samples. Each of the plurality of training samples may include multiple sample black blood images of a sample region and gold standard blood vessel segments corresponding to the sample region.

In some embodiments, for a training sample, the processing device 140 may obtain the sample black blood images of the sample region of the training sample from an imaging device or a storage device. For example, the sample black blood images of the sample region may be obtained in a similar manner as how the black blood images 410 are obtained as described in FIG. 4.

In some embodiments, the gold standard blood vessel segments corresponding to the sample region in the training sample may be determined or confirmed manually and used as a training label. For example, a user may determine the gold standard blood vessel segments corresponding to the sample region via a user terminal that displays the sample black blood images of the sample region.

In some embodiments, each of the plurality of training samples may further include a sample bright blood image of the sample region of the training sample. The sample bright blood image of the sample region may be obtained in a similar manner as how the bright blood image 415 is obtained as described in FIG. 4.

In some embodiments, the training samples are generated based on sample first black blood images and sample second black blood images, wherein the sample first black blood images are acquired using one type of MRI imaging sequence while the sample second black blood images are acquired using another type of MRI imaging sequence. The plurality of training samples may include first training samples and second training samples. Each first training sample may include one of the sample first black blood images and a corresponding copy image, and each second training sample may include one of the sample first black blood images and one of the sample second black blood images that correspond to the sample region. That is, the black blood images in each first training sample are the same, and the black blood images in each second training sample are different.

By designing the first training samples, a single sample black blood image can be used to generate a training sample for training a blood vessel segmentation model, and the blood vessel segmentation model can be used to process a single vascular image, which can expand the application scope of the blood vessel segmentation model.

In 604, the processing device 140 (e.g., the training module 230) may generate the blood vessel segmentation model by training an initial model using the plurality of training samples.

The initial model may be a machine learning model before being trained. Exemplary initial models may include a generative adversarial network (GAN), a U-net, a pixel recurrent neural network (PixelRNN), a draw network, a variational autoencoder (VAE), or the like, or any combination thereof. In some embodiments, the initial model may be a modified machine learning model. For example, the initial model may be a U-net with a residual module. By introducing the residual module, model parameter(s) of the initial model may be optimized, and the efficiency of the model training may be improved.

In some embodiments, the initial model may include multiple input channels, and each of the sample black blood images (and/or the sample bright blood images) may be randomly designated as an input image of one of the input channels during the training process. For example, the initial model may include a first input channel and a second input channel, and sample black blood images of a training sample may include a sample first black blood image and a sample second black blood image. The processing device 140 may designate the sample first black blood image as an input image of the first input channel, and designate the sample second black blood image as an input image of the second input channel, or vice versa.

It should be noted that the initial model and the blood vessel segmentation model are merely provided for illustration purposes, and can be modified according to an actual need. For example, the initial model may include three or more input channels, and each training sample may further include a sample third black blood image or a sample bright blood image. The sample third black blood image may be a black blood image different from the sample first black blood image and the sample second black blood image or a copy image of the sample first black blood image or the sample second black blood image.

By designing more than two input channels, information in different types of images can be comprehensively considered, which can improve the accuracy of the blood vessel segmentation model, thereby further improving the accuracy of the determination of the first segmentation result and the centerline.

In some embodiments, the initial model may be trained according to a machine learning algorithm. For example, the processing device 140 may generate the blood vessel segmentation model according to a supervised machine learning algorithm by performing one or more iterations to iteratively update model parameter(s) of the initial model.

Merely by way of example, the training of the initial model may include an iterative process. The plurality of training samples may be used to iteratively update model parameter(s) of the initial model until a termination condition is satisfied. Exemplary termination conditions may include that a value of a loss function corresponding to the initial model is below a threshold value, a difference of values of the loss function obtained in a previous iteration and the current iteration is within a difference threshold value, a certain count of iterations has been performed, etc. For example, in a current iteration, sample black blood images of a training sample may be input into the initial model, and the initial model may generate a sample concatenation image of the sample black blood images, and generate a prediction result (e.g., a sample first segmentation result or sample blood vessel segments) by processing the sample concatenation image. Then, a value of the loss function may be determined to measure a difference between the prediction result and the training label (e.g., gold standard blood vessel segments in the training sample of the training sample). If it is determined that the termination condition is satisfied in the current iteration, the initial model may be designated as the blood vessel segmentation model; otherwise, the initial model may be further updated based on the value of the loss function.

In some embodiments, a weight value may be designated to each of the multiple input channels of the blood vessel segmentation model. For example, the training samples may be generated based on the sample first black blood images and the sample second black blood images, and the input channels of the blood vessel segmentation model may include the first input channel and the second input channel. A first weight value of the first input channel may be determined based on a proportion of a first count to a total count, and a second weight value of the second input channel may be determined based on a proportion of the second count to the total count. The first count may be a count of the sample first black blood images, the total count may be a sum of the first count and a second count of the sample second black blood images. Merely by way of example, when there are 80 sample first black blood images and 20 sample second black blood images, the processing device 140 may determine the first weight value of the first input channel as 0.8, and the second weight value of the second input channel as 0.2.

As another example, the processing device 140 may determine the weight value corresponding to each of the multiple input channels based on the image quality (e.g., a signal-to-noise ratio (SNR), an image resolution) of the sample first black blood images and the sample second black blood images. As still another example, the weight value corresponding to each of the multiple input channels may be set according to an empirical value.

By automatically determining the weight values corresponding to the multiple input channels, influences of different input channels on the determination of the first segmentation result can be defined, and features of each of the sample black blood images can be comprehensively considered, which can facilitate sharing and integration of information between the input channels, thereby improving the accuracy of the generation of the first segment result. In addition, by introducing the weight values corresponding to the multiple input channels, the training samples for training the blood vessel segmentation model can be generated based on single MR images (e.g., the first training sample including the sample first black blood image and a corresponding copy image), which can expand the scope of the training samples and rich the training samples, thereby improve the performance and robustness of the blood vessel segmentation model. Therefore, the accuracy of the blood vessel segmentation model can be improved, thereby improving the accuracy of the determination of the first segmentation result and the centerline.

Furthermore, by introducing the blood vessel segmentation model, the first segmentation result may be generated automatically, which can improve the efficiency of the determination of the first segmentation result, thereby improving the efficiency of the determination of the centerline. In addition, a corresponding relationship between different types of black blood images (and bright blood images) may be complex. By using the machine learning model (e.g., the blood vessel segmentation model), the analysis of the big data may enable mining the complex corresponding relationship, and realize the accurate determination of the first segmentation result based on different types of black blood images (and bright blood images).

FIG. 7 is a schematic diagram illustrating an exemplary process 700 for determining a centerline of first blood vessels according to some embodiments of the present disclosure.

As illustrated in FIG. 7, a target segment result 470 may be obtained by fusing the first segment result 330 and the second segment result 460, and the centerline 440 of first blood vessels may be determined based on the target segment result 470.

The second segment result 460 may include second blood vessel segments of the first blood vessels in the first region. In some embodiments, the second segment result 460 may be determined by performing a segment operation on a bright blood image (e.g., the bright blood image 450). Exemplary segment operations may include a segment operation based on a threshold segmentation algorithm, a segment operation based on a regional growth algorithm, or the like, or any combination thereof.

In some embodiments, the second segment result 460 may be determined by inputting the bright blood image into a second blood vessel segmentation model. For example, the second blood vessel segmentation model may be the same as or similar to the blood vessel segmentation model as described in FIG. 6. As another example, the second blood vessel segmentation model may be a blood vessel segmentation model for processing a single blood image (e.g., the bright blood image). The second blood vessel segmentation model may be generated by training a second initial model using a plurality of second training samples. Each of the plurality of second training samples may include a sample bright blood image of a sample region and second gold standard blood vessel segments corresponding to the sample region. For a second training sample, the sample bright blood image may be obtained in a similar manner as how the bright blood image is obtained as described above. The second gold standard blood vessel segments may be determined automatically or manually. The second blood vessel segmentation model may be trained in a similar manner as how the blood vessel segmentation model is trained as described in FIG. 6.

The target segment result 470 refers to a segment result used for determining the centerline of the first blood vessels. In some embodiments, the processing device 140 may fuse the first segment result 330 and the second segment result 460 to generate the target segment result 470. For example, the first segment result 330 and the second segment result 460 may be added to generate the target segment result 470. As another example, the processing device 140 may obtain the target segment result 470 by inputting the first segment result 330 and the second segment result 460 into a fusion model.

The fusion model may be a trained machine learning model, which can generate the target segment result by fusing the first segment result and the second segment result. In some embodiments, the fusion model may be generated by training a third initial model using a plurality of third training samples. Each of the plurality of third training samples may include a sample first segment result of a sample black blood image of a sample region, a sample second segment result of a sample bright blood image of the sample region, and a gold standard segment result of the sample region. For a third training sample, the sample first segment result of the sample black blood image may be obtained in a similar manner as how the first segment result is obtained as described above, and the sample second segment result of the sample bright blood image may be obtained in a similar manner as how the second segment result is obtained as described above. The gold standard segment result may be determined automatically or manually. In some embodiments, each of the plurality of third training samples may further include the sample black blood image and the sample bright blood image. The fusion model may be trained in a similar manner as how the blood vessel segmentation model is trained as described in FIG. 6.

In some embodiments, a weight value may be designated to each of the sample first segment result and the sample second segment result. For example, a third weight value corresponding to the sample first segment result and a fourth weight value corresponding to the sample second segment result may be determined according to image quality of the sample black blood image and the sample bright blood image. As another example, the third weight value corresponding to the sample first segment result and the fourth weight value corresponding to the sample second segment result may be determined based on a system default setting or set manually by a user.

In some embodiments, the processing device 140 may determine the centerline 440 of the first blood vessels based on the target segment result 470. For example, the centerline 440 of the first blood vessels may be determined based on the target segment result 470 in a similar manner as how the centerline 440 of the first blood vessels is determined based on the first segment result 330 as described in FIG. 4.

By determining the centerline of the first blood vessels based on the target segment result including the first segment result and the second segment result, features of both the black blood images and the bright blood image(s) can be comprehensively considered, thereby improving the accuracy of the determination of the centerline.

In some embodiments, the processing device 140 may determine a first centerline 710 of the first blood vessels by performing a centerline extraction operation on the target segment result 470, and obtain a second centerline 720 of the first blood vessels by performing an endpoint culling operation on the first centerline 710 of the first blood vessels. Further, the processing device 140 may determine the centerline 440 of the first blood vessels by correcting, based on the target segment result 470, the second centerline 720 of the first blood vessels.

The first centerline 710 may be determined in a similar manner as how the centerline 440 of the first blood vessels is determined based on the first segment result 330 as described in FIG. 4. For example, the processing device 140 may determine a sub-centerline of each blood vessel segment in the target segment result 470 by performing the centerline extraction operation on the blood vessel segment using an optimum route algorithm, and determine the first centerline 710 by connecting the sub-centerline of each blood vessel segment based on connected components in the target segment result 470.

Since the adhesion exists between endpoints of the blood vessel segments, redundant endpoints may appear around the adhesion, which results in winding and reduces the accuracy of the centerline. To reduce or eliminate the influence of the adhesion on the determination of the centerline, the processing device 140 may perform the endpoint culling operation on the first centerline 710 of the first blood vessels. Taking cerebrovascular as an example, a first centerline may be generated by connecting a sub-centerline of each blood vessel segment corresponding to connected components of the internal carotid artery and the vertebral artery in a black blood image (e.g., the black blood image 302). Due to obstructed blood vessels in the internal carotid artery and the vertebral artery, the first centerline may be broken into a plurality of centerline segments. The processing device 140 may retain a portion of the first centerline (e.g., a certain count of the plurality of centerline segments), and perform the endpoint culling operation on endpoints of each of the portion of the first centerline, so as to generate the second centerline 720.

In some embodiments, the processing device 140 may determine the portion of the first centerline (e.g., retained centerline segments) according to an area threshold or a length threshold. For example, the processing device 140 may determine the area threshold (or the length threshold) according to an empirical value, and determine the retained centerline segments by comparing an area (or a length) of each of the plurality of centerline segments with the area threshold (or the length threshold). In some embodiments, the processing device 140 may determine the portion of the first centerline based on an area sequence (or a length sequence). For instance, the processing device 140 may sort the plurality of centerline segments based on the area (or the length) of each of the plurality of centerline segments, and determine the retained centerline segments based on a preset count of centerline segments and the area sequence (or the length sequence) of the plurality of centerline segments. The preset count of centerline segments may be determined based on a system default setting or set manually by a user.

By performing the endpoint culling operation, the adhesion can be removed from the first centerline, which can reduce errors in the determination of the centerline and improve the accuracy of the determination of the centerline.

In some embodiments, the processing device 140 may correct the second centerline 720 of the first blood vessels based on the target segment result 470 to determine the centerline 440 of the first blood vessels. For example, the processing device 140 may determine at least one bifurcation point 730 in the target segment result 470 based on the second centerline 720 of the first blood vessels, and determine the centerline 440 of the first blood vessels based on the at least one bifurcation point 730.

A bifurcation point refers to a position where a first blood vessel bifurcates.

Since a position of each endpoint of the first blood vessels may be shifted during the determination of the centerline (e.g., the centerline extraction operation), a distance difference between a determined position of the endpoint and an actual position of the endpoint may exist. By determining the at least one bifurcation point 730 based on the second centerline 720 generated after the endpoint culling operation, the shift of the endpoints can be reduced or eliminated, which can improve the accuracy of the determination of the at least one bifurcation point 730.

In some embodiments, the at least one bifurcation point 730 may be determined based on the second centerline 720 using a vascular branch curvature analysis algorithm, etc.

In some embodiments, the processing device 140 may further determine a third centerline 740 of the first blood vessels based on the at least one bifurcation point 730, and determine the centerline 440 of the first blood vessels by correcting the third centerline 740 of the first blood vessels. The correction on the third centerline 740 may include correcting a position of the third centerline 740 in the target segment result 470, expending the third centerline 740 based on the second segment result 460, etc.

For example, the processing device 140 may obtain vascular wall information 750 of the first blood vessels based on at least one of the multiple black blood images, and determine the centerline 440 of the first blood vessels by correcting the third centerline 740 of the first blood vessels based on the vascular wall information. For example, the processing device 140 may determine a position of the third centerline 740, and compare the position of the third centerline 740 with the vascular wall information 750 (e.g., a position of the vascular wall). If it is determined that the position of the third centerline 740 is not located at a center of the vascular wall according to the comparison result, the processing device 140 may correct the position of the third centerline 740 based on the position of the vascular wall. The vascular wall information 750 may be determined by manually labeling the at least one of the multiple black blood images. Alternatively, the vascular wall information 750 may be determined using an image identification algorithm, a feature extraction algorithm based on a neural network, etc.

As another example, the processing device 140 may obtain the second segment result 460, and determine the centerline 440 of the first blood vessels by performing an extension operation on the third centerline 740 of the first blood vessels based on the second segment result 460.

Since a length of at least one of second blood vessel segments in the second segment result 460 is larger than a length of the corresponding first blood vessel segment(s) in the first segment result 330 or a length of corresponding target blood vessel segment(s) in the target segment result 470, the extension operation may be performed on the third centerline 740 of the first blood vessels based on the second segment result 460. For example, at least one seed point may be determined in the third centerline 740 based on the second segment result 460. A seed point refers to an original of a centerline segment whose length is less than a length of the corresponding second blood vessel segment in the second segment result and that needs to be expanded. For each of the at least one seed point, the processing device 140 may extend a centerline segment in the third centerline 740 corresponding to the seed point upward and/or downward for a certain distance from the seed point, until the centerline segment is consistent with an actual length of the corresponding second blood vessel segment in the second segment result 460. Therefore, the third centerline 740 may be extended to be consistent with an actual total length of the second blood vessel segments in the second segment result 460.

By determining the third centerline 740 based on the at least one bifurcation point 730, correcting the third centerline 740 based on the vascular wall information 750 and/or the second segment result 460, etc., an accurate centerline 440 can be generated by performing multifaceted corrections.

It should be noted that the determination of the centerline is merely provided for illustration purposes, and can be modified according to an actual need. For example, the processing device 140 may obtain the first centerline 710 of the first blood vessels by performing the centerline extraction operation on the first segment result 330, obtain the second centerline 720 of the first blood vessels by performing the endpoint culling operation on the first centerline 710 of the first blood vessels, and determine the centerline 440 of the first blood vessels by correcting, based on the target segment result 470, the second centerline 720 of the first blood vessels.

FIG. 8 is a schematic diagram illustrating an exemplary process 800 for determining a centerline of first blood vessels according to some embodiments of the present disclosure.

As illustrated in FIG. 8, a first black blood image (e.g., a T1 image 810) and a second black blood image (e.g., a T1CE image 820) of a first region of a target subject may be input into multiple input channels of a blood vessel segmentation model. The blood vessel segmentation model may generate a concatenation image 830 of the T1 image 810 and the T1CE image 820, and generate a first segment result 840 (blood vessel segments of first blood vessels in the first region) by processing the concatenation image 830. The processing device 140 may further obtain a bright blood image 850 of the first region of the target subject, and obtain a second segment result 860 (second blood vessel segments of the first blood vessels in the first region) corresponding to the bright blood image 850 by performing segment operation on the bright blood image 850. The processing device 140 may obtain a target segment result 870 by fusing the first segment result 840 and the second segment result 860, and determine a centerline 880 of the first blood vessels based on the target segment result 870.

FIG. 9A is a flowchart illustrating an exemplary process 900A for generating a target image according to some embodiments of the present disclosure.

To diagnose and/or treat vascular diseases, imaging technology is used for navigation and/or guidance of vascular paths. For example, a DSA technique can be used to assess the degree of vascular stenosis and detect the location of vascular blockages. However, since the obstructed blood vessels cannot be filled with a contrast agent, the DSA technique cannot distinguish the composition of the obstructed blood vessels, which limits its ability to provide precise vascular path navigation. In order to solve the above problem, the process 900A may be performed.

As illustrated in FIG. 9A, the processing device 140 may obtain the first image 310 and the second image 340 of a target subject. The first image 310 may indicate the vascular wall in a first region of the target subject, and the second image 340 may indicate the vascular lumen in a second region of the target subject. The first region and the second region may both include a target region of the target subject.

The first image 310 may include an image including image information regarding the vascular wall. For example, the first image 310 may be a black blood image including image information of obstructed tissues and non-obstructed tissues of the vascular wall. Since the vascular lumen is surrounded by the vascular wall, the first image 310 may further include image information regarding the vascular lumen in the first region.

In some embodiments, the first image 310 may be one of the multiple black blood images as described in FIGs. 3 and 4.

In the first image, the surrounding tissues (e.g., the vascular wall) of first blood vessels in the first region and lesions or abnormalities (e.g., the obstructed tissues, plaques, clots, or other structural issues) within or adjacent to the first blood vessels can be presented clearly. In this way, more image information of the first blood vessels (including the vascular wall and the vascular lumen) can be provided, thereby improving the accuracy of the analysis on the first blood vessels.

The second image 340 may be an image acquired using a DSA technique. The DSA technique refers to a fluoroscopy technique used in interventional radiology to clearly visualize blood vessels. The DSA technique may have advantages, such as, high contrast resolution, short imaging time, less use of contrast agent, accurately positioning the narrowing of blood vessels, etc.

In some embodiments, the processing device 140 may obtain the first image 310 before acquiring the second image 340. For example, when the second image 340 is acquired using the DSA technique, the processing device 140 may retrieve the first image 310 from an imaging device (e.g., the imaging device 110, an MRI device, an MRI scanner of a multi-modality imaging device, etc.) or a storage device (e.g., the storage device 150, a database, or an external storage) that stores the first image 310.

The processing device 140 may generate a registration result 345 by registering the first image 310 and the second image 340.

In some embodiments, the registration result 345 may include registration information between the first image 310 and the second image 340. The registration information may indicate a corresponding relationship between first pixels (or voxels) in the first image 310 and second pixels (or voxels) in the second image 340 and/or a transformation relationship between first coordinates of the first pixels (or voxels) in the first image 310 and second coordinates of the second pixels (or voxels) in the second image 340. For example, each physical point of the target subject may correspond to a first pixel in the first image 310 and a second pixel in the image 340, wherein the first pixel and the second pixel may be regarded as being corresponding to each other. As another example, for each physical point of the target subject, a first coordinate of the corresponding first pixel in the first image 310 and a second coordinate of the corresponding second pixel in the second image 340 may be transformed into each other through the transformation relationship.

In some embodiments, the registration information may be represented by a transformation matrix or a motion field between the first image 310 and the second image 340. The motion field refers to a function representing the corresponding relationship and/or the transformation relationship between the first pixels (or voxels) in the first image 310 and the second pixels (or voxels) in the second image 340.

In some embodiments, the processing device 140 may determine the registration information between the first image 310 and the second image 340 using an image registration algorithm. Exemplary image registration algorithms may include a grayscale-based registration algorithm, a domain-based registration algorithm, a feature-based registration algorithm, a point-based registration algorithm (e.g., an anatomic-landmark-based registration algorithm), a curve-based registration algorithm, a surface-based registration algorithm (e.g., a surface-profile-based surface profile), a spatial alignment registration algorithm, a cross-correlation registration algorithm, a mutual-information-based registration algorithm, a sequential similarity detection algorithm (SSDA), a nonlinear transformation registration algorithm, an optical flow, demons registration algorithm, B-spline registration algorithm, or the like, or any combination thereof.

Merely by way of example, the processing device 140 may determine the first coordinates of the first pixels (or voxels) in the first image 310 and the second coordinates of the second pixels (or voxels) in the second image 340, and perform a rigid registration between the first image 310 and the second image 340, so as to determine the corresponding relationship between the first pixels (or voxels) in the first image 310 and the second pixels (or voxels) in the second image 340. Correspondingly, the processing device 140 may designate the corresponding relationship as the registration result 345.

By determining the registration result 345, tissues in the first image 310 and the second image 340 can be mapped, which facilitates the subsequent presentation of the first image 310 and the second image 340.

The processing device 140 (e.g., the generation module 220) may generate the target image 350 based on the registration result 345, the first image 310, and the second image 340.

The target image 350 refers to an image for presentation and/or analysis. For example, the target image 350 may include image information regarding the target region (e.g., blood vessels in the target region) for presenting to a user (e.g., a doctor, a technician, etc.).

In some embodiments, the processing device 140 may generate the target image 350 by combining the first image 310 and the second image 340 based on the registration result 345. For example, the processing device 140 may generate the target image 350 by displaying the first image 310 and the second image 340 in parallel based on the registration result 345. As another example, the processing device 140 may generate the target image 350 by fusing the first image 310 and the second image 340 based on the registration result 345.

In some embodiments, the processing device 140 may display the target image 350 based on the first blood vessels or the second blood vessels. For example, the processing device 140 may display a portion of the target image 350 according to vascular paths of the first blood vessels.

In conventional DSA techniques, only the second image, which shows the vascular lumen, is displayed to the user. This approach does not reveal the vascular walls, which can impair intraoperative navigation during the DSA scanning process. By incorporating the first image along with the second image, both the vascular lumen and the vascular walls are presented, enhancing the visibility of vascular paths and significantly improving intraoperative navigation.

In some embodiments, the second region may be part of the first region, the processing device 140 may further determine a local image 910 in the first image 310 corresponding to the second image 340 based on the registration result 345, and generate the target image 350 based the local image 910 and the second image 340.

The local image 910 may be a portion of the first image 310 corresponding to the second image 340 (or a portion of the second image 340). For example, the local image 910 may correspond to the target region in the second image 340.

The target image 350 may include the local image 910 and the second image 340 displayed in parallel, or the target image 350 may be a fusion image of the local image 910 and the second image 340.

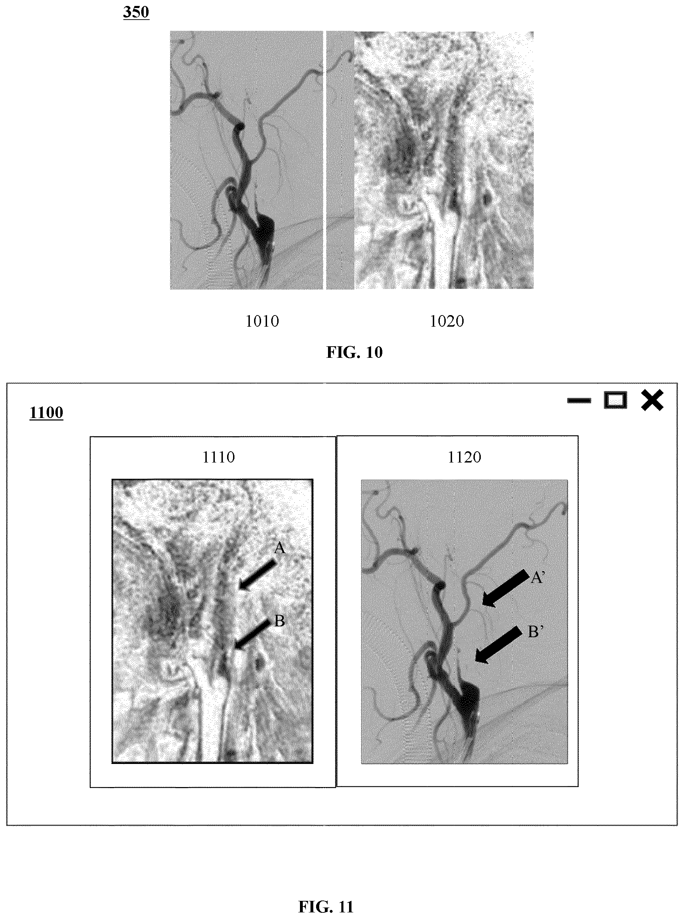

Merely by way of example, referring to FIG. 10, FIG. 10 is a schematic diagram illustrating an exemplary target image 350 according to some embodiments of the present disclosure. As illustrated in FIG. 10, the target image 350 may include a left image 1010 and a right image 1020, the left image 1010 may be a DSA image (e.g., the second image 340), and the right image 1020 may be a local image of a black blood image (e.g., the first image 310). The right image 1020 may be determined based on a registration result between the left image 1010 and the black blood image. For example, the left image 1010 and the right image 1020 may be displayed in parallel, so as to generate the target image 350. The left image 1010 may present the vascular lumen of blood vessels in the target region, and the right image 1020 may present the vascular wall of the blood vessels in the target region. Therefore, the target image 350 may present information regarding the vascular lumen of the blood vessels and information regarding the vascular wall of the blood vessels (including obstructed tissues).

By providing the target image, more vascular information (e.g., the information regarding the vascular wall of the blood vessels (including obstructed tissues)) can be provided, which can improve the accuracy of the navigation of the blood vessels, thereby improving the accuracy of the vascular detection and/or analysis.

In some embodiments, the processing device 140 may further determine blood vessels of interest in the first image 310 corresponding to the blood vessels of interest in the second image 340 based on the registration result 345, and generate the target image 350 that includes the first image 310 and the second image 340 displayed in parallel and includes a label corresponding to the blood vessels of interest in the first image 310.

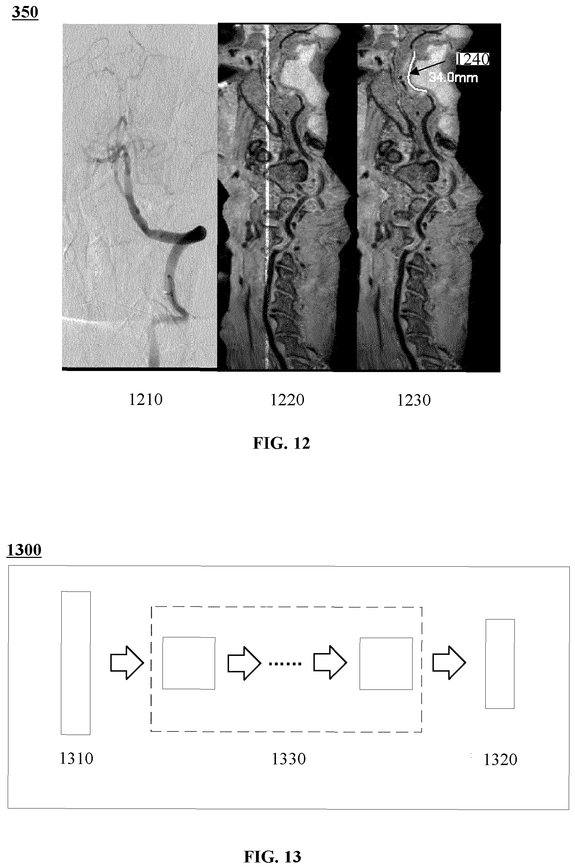

Merely by way of example, referring to FIG. 12, FIG. 12 is a schematic diagram illustrating an exemplary target image 350 according to some embodiments of the present disclosure. As illustrated in FIG. 12, the target image 350 may include a left image 1210, a middle image 1220, and a right image 1230. The left image 1210 may be a DSA image (e.g., the second image 340), the middle image 1220 may be a black blood image (e.g., the first image 310), and the right image 1230 may be the middle image 1220 including a label 1240 corresponding to blood vessels of interest in the left image 1210.

By generating the target image including the label corresponding to the blood vessels of interest in the first image, the information regarding the vascular lumen and the information regarding the vascular wall can be combined and presented in a same image, which can provide more information to the user and facilitate the user diagnosis and analysis.

In some embodiments, the processing device 140 may further perform an abnormality analysis on the target image 350 and/or the first image 310. For example, the processing device 140 may input the target image 350 into a plaque determination model, and the plaque determination model may output a plaque determination result. The plaque determination result may include whether a plaque exists in the target image 350, a position of the plaque exists in the target image 350, etc.

In some embodiments, the plaque determination model may be a trained machine learning model. In some embodiments, the plaque determination model may include a multi-layer structure. Merely by way of example, as illustrated in FIG. 13, FIG. 13 is a schematic diagram illustrating an exemplary plaque determination model 1300 according to some embodiments of the present disclosure. The plaque determination model 1300 may include an input layer 1310, an output layer 1320, and one or more convolution layers 1330 between the input layer 1310 and the output layer 1320. The target image 350 and/or the first image 310 may be input into the input layer 1310 of the plaque determination model 1300, and the output layer 1320 of the plaque determination model 1300 may output the plaque determination result. In some embodiments, the input layer 1310 and the one or more convolution layers 1330 may be connected through a convolution computing unit, a batch normalization unit, a linear correction unit, a maximum pooling unit, etc., and the one or more convolution layers 1330 and the output layer 1320 may be connected through a threshold processing unit, a key point output unit, a region size output unit, etc.

In some embodiments, the processing device 140 may obtain the plaque determination model from a storage device (e.g., the storage device 150) of the imaging system 100 or a third-party database. In some embodiments, the plaque determination model may be generated by the processing device 140 or another computing device according to a machine learning algorithm. In some embodiments, the plaque determination model may be generated by a computing device (e.g., the processing device 140) by training a fourth initial model using a plurality of fourth training samples for generating the plaque determination model disclosed herein. Each of the plurality of fourth training samples may include a sample image of a sample region and a gold standard plaque determination result (e.g., sample positioning information of a sample plaque in the sample region). For a fourth training sample, the sample image of the sample region may be obtained in a similar manner as how the target image 350 and/or the first image 310 is obtained as described above. The gold standard plaque determination result may be determined automatically or manually.

In some embodiments, the processing device 140 may generate a real-time path of the blood vessels of interest based on the first image 310, the second image 340, and the registration result 345. In such case, vascular paths of the blood vessels of interest can be presented synchronously with the first image 310 and the second image 340. That is, during the DSA scanning process, the user can check the first image 310 and the second image 340 simultaneously, which can result in accurate and complete navigation for the blood vessels of interest.

In some embodiments, the processing device 140 may obtain the first image 310 of the target subject. The first image 310 may indicate the vascular wall in the first region of the target subject. The processing device 140 may obtain the second image 340 of the target subject. The second image 340 may indicate the vascular lumen in the second region of the target subject, and the first region and the second region may both include the target region of the target subject. The processing device 140 may generate the registration result 345 by registering the first image 310 and the second image 340, and generate the target image 350 based on the registration result 345, the first image 310, and the second image 340.

In some embodiments, the processing device 140 may generate a first extraction result of the first blood vessels in the first region by extracting the vascular wall from the first image, obtain a second extraction result of second blood vessels in the second region by extracting the vascular lumen from the second image, and determine the target image 350 based on the registration result 345, the first extraction result, and the second extraction result.

Merely by way of example, referring to FIG. 9B, FIG. 9B is a flowchart illustrating an exemplary process 900B for generating a target image according to some embodiments of the present disclosure.

As illustrated in FIG. 9B, the processing device 140 may generate a first extraction result 920 of the first blood vessels in the first region by extracting (or referred to as segmenting) the vascular wall from the first image 310, and obtain a second extraction result 930 of second blood vessels in the second region by extracting the vascular lumen from the second image 340

The first extraction result 920 may indicate the vascular wall and the vascular lumen (since the vascular wall is surrounded by the vascular wall) extracted from the first image 310. In some embodiments, the first extraction result 920 may include other information determined based on the vascular wall and the vascular lumen extracted from the first image 310. For example, the first extraction result 920 may include a centerline of the first blood vessels, vascular parameters of the first blood vessels, etc. Exemplary vascular parameters may include information regarding the vascular wall, information regarding the vascular lumen, information regarding vascular components (e.g., an area, an area proportion, a load parameter, etc., of each vascular component), or the like, or any combination thereof.

The second extraction result 930 may indicate the vascular lumen extracted from the second image 340. In some embodiments, the second extraction result 930 may include other information determined based on the vascular lumen extracted from the first image 310. For example, the second extraction result 930 may include a centerline of the second blood vessels, vascular parameters of the second blood vessels (e.g., information regarding the vascular lumen), etc. For instance, the processing device 140 may obtain an enhancement image by performing an image enhancement on the second image 340, and obtain the second extraction result 930 by extracting the vascular lumen based on a threshold segmentation algorithm and a path tracing algorithm. As another example, the processing device 140 may obtain the second extraction result 930 using an image segmentation algorithm, a filtering algorithm, a machine learning algorithm, etc.

Further, the processing device 140 may determine the target image 350 based on the registration result 345, the first extraction result 920, and the second extraction result 930. For example, the processing device 140 may determine the target image 350 by reconstructing the target region based on the registration result 345, the first extraction result 920, and the second extraction result 930. As another example, the processing device 140 may generate comparison information between the first image 310 and the second image 340 based on the registration result 345, the first extraction result 920, and the second extraction result 930, and determine the comparison information as the target image 350.

Merely by way of example, Referring to FIG. 11, FIG. 11 is a schematic diagram illustrating an exemplary presentation interface 1100 according to some embodiments of the present disclosure. As illustrated in FIG. 11, a presentation interface 1100 may be configured to present a target image including a left image 1110 and a right image 1120 displayed in parallel. The target image may be generated based on a registration result, a first extraction result, and a second extraction result. For example, the first extraction result and the second extraction result may be synchronously presented in the left image 1110 and the right image 1120, respectively, based on the registration result 345. For instance, arrows A and B in the left image 1110 may be used to label obstructed blood vessels in the first extraction result, and arrows A' and B' in the right image 1120 may be used to label corresponding obstructed blood vessels in the second extraction result based on the registration result 345.

By presenting the comparison information between the first image and the second image, the accuracy of the navigation and/or guidance of the vascular paths in the blood vessels of interest can be improved. It should be noted that the presentation interface is merely provided for illustration purposes, and is not intended to limit the scope of the present disclosure. For example, the presentation interface may be configured to present other information, such as, a length of the obstructed blood vessels, etc.

As another example, the processing device 140 may generate fusion information of the first image 310 and the second image 340 based on the registration result 345, the first extraction result 920, and the second extraction result 930. For instance, the processing device 140 may generate the target image 350 by fusing the first extraction result 920 and the second extraction result 930 based on the registration result 345. In this case, the target image 350 may include information relating to both the vascular wall and the vascular lumen in the blood vessels of interest.

In some embodiments, the processing device 140 may further perform an abnormity analysis on the first image 310, and present the abnormity determination result together with the target image 350. By presenting the above data, rich information about the blood vessels can be presented to the user, which can help the user understand the status of the blood vessels clearly and comprehensively.

According to some embodiments of the present disclosure, the registration result can be generated by registering the first image indicating the vascular wall and the second image indicating the vascular lumen, and the target image can be generated based on the registration result, the first image, and the second image. Therefore, more information regarding the obstructed blood vessels and the surrounding tissues can be provided and presented to the users, which can improve the accuracy of the analysis of the blood vessels and the navigation of the vascular paths.

FIG. 14 is a flowchart illustrating an exemplary process 1400 for determining at least one abnormal vascular region according to some embodiments of the present disclosure.

In the imaging analysis of cerebrovascular, a single imaging analysis manner is used to independently analyze individual image information. For example, structural image analysis (e.g., a vascular analysis, a plaque detection, etc.) is performed alone to analyze structural image information, and functional information analysis is performed alone to analyze functional information in the brain region. Consequently, the analysis and detection results of medical imaging data are often limited by single information sources, leading to lower accuracy in detecting abnormal blood vessels. In order to solve the above problem, the process 1400 may be performed.

As illustrated in FIG. 14, in some embodiments, first detection information 370 of a target region may be determined by processing the first image 310 (e.g., at least one of multiple black blood images), and second detection information 375 of the target region may be determined by processing the third image 360 of a third region of the target subject. In some embodiments, the first image 310 may be any structural image, such as, CT image, MRI image, etc., and the third image 360 may be any functional image, such as, a perfusion image, a PET image, etc.

The first detection information 370 refers to a structural analysis result of the first image 310. In some embodiments, the first detection information 370 may include information similar to the first extraction result 920.

In some embodiments, the processing device 140 may obtain the first detection information 370 by performing a structural analysis on the first image 310. Alternatively, the first detection information 370 may be determined in a similar manner as how the first extraction result 920 is determined as described in FIG. 9B.

The third region may include the target region.

In some embodiments, the third image 360 may be acquired using a perfusion imaging technique (e.g., a perfusion imaging sequence). The perfusion imaging technique refers to a technique used to evaluate the microcirculation and hemodynamics state of a target subject. For example, the perfusion imaging technique may be used to measure neuronal activity, such as, microcirculation and hemodynamics of the neuronal activity.

The second detection information 375 refers to a functional analysis result of the third image 360. For example, the second detection information 375 may include perfusion parameters, such as, a cerebral blood flow (CBF), a cerebral blood volume (CBV), a mean transit time (MTT), a time-to-peak (TTP) of a concentration of the contrast agent, a time-to-maximum (Tmax) of a residual amount of the contrast agent, or the like, or any combination thereof.

In some embodiments, the processing device 140 may obtain the second detection information 375 by performing a functional analysis on the third image 360. For example, the processing device 140 may determine the second detection information 375 using a perfusion processing model.

Further, at least one abnormal vascular region 380 in the target region may be determined based on the first detection information 370 and the second detection information 375.

In some embodiments, the at least one abnormal vascular region 380 may include a plaque in the target region. For example, the first detection information 370 may include abnormal plaque information, the second detection information 375 may include abnormal functional information, and the at least one abnormal vascular region 380 may be determined based on the abnormal plaque information, the abnormal functional information, and blood supply relationship information corresponding to the target region.

The abnormal plaque information may include abnormal plaques identified from the first image 310 in the structural analysis, such as plaques having erosion, rupture, calcification, etc. The abnormal functional information may include tissues that appear abnormal identified from the third image 360 in the functional analysis. The blood supply relationship information refers to information regarding a corresponding relationship between tissues and blood vessels.

In some embodiments, the first detection information 370 may include at least one structural feature, the second detection information 375 may include at least one functional feature, and the at least one abnormal vascular region may be determined based on the at least one structural feature and the at least one functional feature.

The at least one structural feature may include a calcification feature, a lipid core feature, a standardized vascular wall index, or the like, or any combination thereof. The at least one functional feature may include the CBF, the CBV, the MTT, the TTP, the Tmax, a mismatch of an activation mode, or the like, or any combination thereof.

By combining the first detection information and the second detection information, the structural feature(s) and the functional feature(s) can be used to determine the abnormal vascular region(s) simultaneously, which can improve the accuracy of the determination of abnormal vascular region(s).

In some embodiments, the processing device 140 may determine the at least one abnormal vascular region 380 based on the first detection information 370 and the second detection information 375 using an abnormal information determination model 1410. For example, as illustrated in FIG. 14, the first detection information 370 and the second detection information 375 may be input into the abnormal information determination model 1410, and the abnormal information determination model 1410 may output the at least one abnormal vascular region 380.

The abnormal information determination model 1410 refers to a process or an algorithm for determining the at least one abnormal vascular region 380 based on the first detection information 370 and the second detection information 375. For example, the abnormal information determination model 1410 may be a probability model. The probability model may be configured to determine a probability that each candidate plaque in the target region is a plaque of responsibility. The candidate plaque(s) in the target region may be determined based on the first detection information 370 (e.g., the abnormal plaque information). Alternatively, the candidate plaque(s) in the target region may be determined using a plaque determination model (e.g., the plaque determination model as described in FIG. 9A).

Merely by way of example, the probability model may be represented according to Equation (2)

$$p = x * \sum_{i=1}^{n} ri * ai + y * \sum_{j=1}^{m} wj * bj, \qquad (2)$$

where p refers to a probability corresponding to a candidate plaque in the target region, n refers to a count of the at least one structural feature, m refers to a count of the at least one functional feature, $ai$ refers to a score value of an i-th structural feature in the at least one structural feature, $ri$ refers to a weight value of the i-th structural feature, $bj$ refers to a score value of a j-th functional feature in the at least one functional feature, $wj$ refers to a weight value of the j-th functional feature, $x$ refers to a weight value of the structural analysis, and $y$ refers to a weight value of the functional analysis.

The weight value of the i-th structural feature may be determined based on an importance of the structural feature in the identification of the abnormal vascular region. For example, for a calcification plaque, the calcification feature has the highest importance. Correspondingly, the calcification feature may be determined as a highest weight value. Similarly, the weight value of the j-th functional feature may be determined based on the importance of the functional feature in the identification of the abnormal vascular region .

Merely by way of example, a weight value of each structural feature and each functional feature may be predetermined according to an actual condition, and then the at least one abnormal vascular region 380 may be determined based on the weight value and the score value of each of the at least one structural feature and the at least one functional feature using the abnormal information determination model 1410. For instance, the weight value and the score value of each of the at least one structural feature and the at least one functional feature may be determined as shown in Tables 1 and 2, and the at least one abnormal vascular region 380 may be determined based on Tables 1 and 2 using the abnormal information determination model 1410.

Table 1. Weight values and Score Values of Structural Features

| Weight value | Score value | Structural Feature |
|---|---|---|
| r1 | a1 | Calcification feature |
| r2 | a2 | Lipid core feature |
| r3 | a3 | Standardized vascular wall index |
| ... | ... | ... |

Table 2. Weight values and Score values of Functional Features

| Weight value | Score value | Functional Feature |
|---|---|---|
| w1 | b1 | CBF |
| w2 | b2 | CBV |
| w3 | b3 | mismatch of activation mode |
| ... | ... | ... |

In some embodiments, the processing device 140 may determine the at least one abnormal vascular region 380 by comparing the probability of each candidate plaque with a first probability threshold. For example, if a probability of a candidate plaque is larger than the first probability threshold, the processing device 140 may determine the candidate plaque as a plaque of responsibility (i.e., one of the at least one abnormal vascular region 380). The first probability threshold may be determined based on a system default setting or set manually by a user. For example, the first probability threshold may include 50%, 60%, 70%, 80%, 90%, 95%, etc.

As another example, the abnormal information determination model 1410 may be a trained machine learning model. For example, the abnormal information determination model 1410 may include a machine learning-based classification model, such as a decision tree, an artificial neural network model, a multi-layer perception machine, a KNN, a support vector machine (SVM), a simple Bayes model, an Adaboost model, a logic regression model, a random forest, a gradient boost tree, a gradient boosted decision tree (GBDT), etc.

The abnormal information determination model 1410 may obtain a determination result based on the first detection information 370 and the second detection information 375, and further determine the at least one abnormal vascular region 380 based on the determination result. The determination result may indicate whether each candidate plaque is a plaque of responsibility. For example, the determination result of each candidate plaque may include 1 or 0. The value "1" may indicate that the candidate plaque is a plaque of responsibility, and the value "0" may indicate that the candidate plaque is not a plaque of responsibility. Then, the abnormal information determination model 1410 may determine the candidate plaque(s) of 1 as the at least one abnormal vascular region 380. As another example, the determination result of each candidate plaque may include a probability that the candidate plaque is a plaque of responsibility, and then the candidate plaque(s) each of whose probability is larger than a second probability threshold may be determined as the at least one abnormal vascular region 380. The second probability threshold may be determined based on a system default setting or set manually by a user. In some embodiments, the second probability threshold may be the same as or different from the first probability threshold.

In some embodiments, the abnormal information determination model 1410 may be obtained by training an initial model (also referred to as a fifth initial model) based on a plurality of training samples 1440 (also referred to as a plurality of fifth training samples). In some embodiments, each of the plurality of training samples 1440 may include sample first detection information 1442, sample second detection information 1444, and a sample label 1446. The sample first detection information 1442 may be obtained in a similar manner as how the first detection information 370 is obtained as described above, and the sample second detection information 1444 may be obtained in a similar manner as how the second detection information 375 is obtained as described above. The sample label 1446 may include gold standard abnormal vascular region(s). In some embodiments, the sample label 1446 may be determined automatically or manually. For example, a user may determine the gold standard abnormal vascular region(s) based on the sample first detection information 1442 and the sample second detection information 1444.

According to some embodiments of the present disclosure, by combining the structural feature(s) and the functional feature(s), and determining the weight values of the structural feature(s) and the functional feature(s), the accuracy of the determination of the at least one abnormal vascular region 380 can be improved.

In some embodiments, the processing device 140 may determine a second registration result 1420, and determine a second target image 1430 based on the second registration result 1420. The second registration result 1420 may include registration information between the first image 310 and the third image 360. The second target image 1430 may include the first image 310 (or a local image in the first image 310 corresponding to the target region) and the third image 360 (or a local image in the third image 360 corresponding to the target region) displayed in parallel. Alternatively, the second target image 1430 may be a fusion image of the first image 310 (or a local image in the first image 310 corresponding to the target region) and the third image 360 (or a local image in the third image 360 corresponding to the target region).

In some embodiments, the second registration result 1420 may be determined in a similar manner as how the registration result 345 is determined, and the second target image 1430 may be determined in a similar manner as how the target image 350 is determined. In some embodiments, the processing device 140 may further present the second target image 1430.

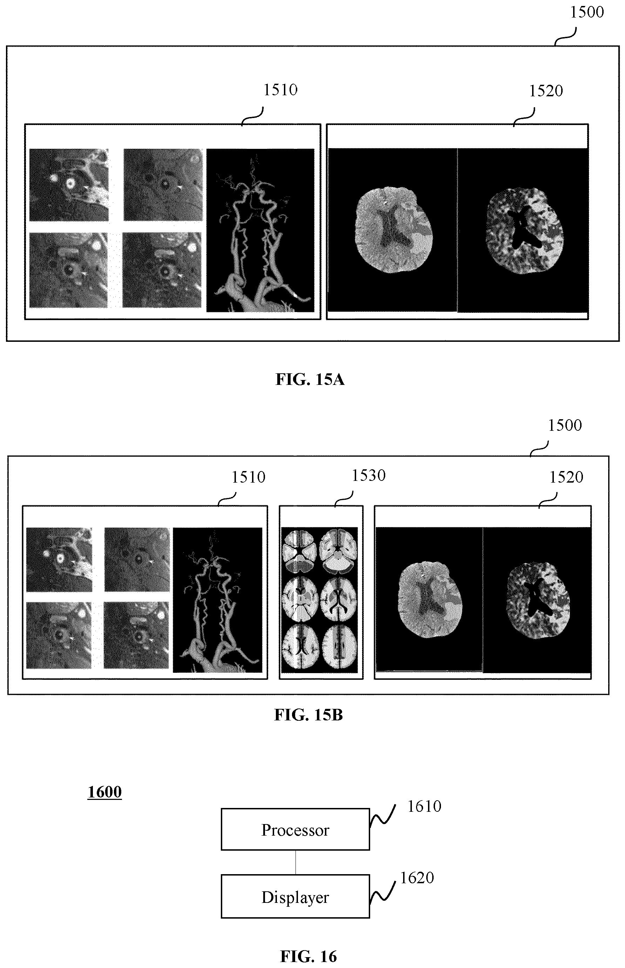

For example, Referring to FIG. 15A, FIG. 15A is a schematic diagram illustrating an exemplary presentation interface 1500 according to some embodiments of the present disclosure. As illustrated in FIG. 15A, a presentation interface 1500 may include a first presentation region 1510 and a second presentation region 1520. The first presentation region 1510 may be configured to present a local image in the first image 310 corresponding to the target region, and the second presentation region 1520 may be configured to present a local image in the third image 360 corresponding to the target region based on the second registration result 1420.

In some embodiments, the processing device 140 may present the second target image 1430 and the blood supply relationship information corresponding to the target region. For example, Referring to FIG. 15B, the presentation interface 1500 may further include a third presentation region 1530 located between the first presentation region 1510 and the second presentation region 1520. The third presentation region 1530 may be configured to present the blood supply relationship information corresponding to the target region.

It should be noted that the presentation interfaces in FIG. 15A and FIG. 15B are merely provided for illustration purposes, and are not intended to limit the scope of the present disclosure. For example, the first presentation region 1510 and the second presentation region 1520 may have an arrangement different from FIG. 15A, such as, an up and down arrangement, an arrangement that scales one of the first presentation region 1510 and the second presentation region 1520 into the other of the first presentation region 1510 and the second presentation region 1520, etc.

By presenting the second target image (and the blood supply relationship information), the first image and the third image can be presented jointly, which can provide rich structural analysis information (e.g., the first detection information) and functional analysis information (e.g., the second detection information) on the same presentation interface, so as to facilitate the user to process subsequent data analysis and processing against the structural analysis information and functional analysis information, thereby improving the efficiency of the vascular detection.

In some embodiments, the processing device 140 may further generate a third target image by combing the first image 310, the second image 340, and the third image 360 based on the registration result 345 and the second registration result 1420. The third target image may be generated in a similar manner as how the target image 350 and/or the second target image 1430 are generated.

In some embodiments, the processing device 140 may obtain the first image 310 of the first region of the target subject, and obtain the third image 360 of the third region of the target subject. The first region and the third region may include the target region. The processing device 140 may determine the first detection information 370 of the target region by processing the first image 310, and determine the second detection information 375 of the target region by processing the third image 360. Further, the processing device 140 may determine the at least one abnormal vascular region 380 in the target region based on the first detection information 370 and the second detection information 375.

According to some embodiments of the present disclosure, the structural analysis information (e.g., the first detection information) and the functional analysis information (e.g., the second detection information) can be combined to provide rich information for determining the at least one abnormal vascular region, thereby improving the accuracy of the vascular detection.

It should be noted that the descriptions of the processes 300, 400, 600, 700, 900A, 900B, and 1400 are provided for the purposes of illustration, and are not intended to limit the scope of the present disclosure. For persons having ordinary skills in the art, various variations and modifications may be conducted under the teaching of the present disclosure. For example, the processes 300, 400, 600, 700, 900A, 900B, and 1400 may be accomplished with one or more additional operations not described, and/or without one or more of the operations discussed. Additionally, the order in which the operations of the processes 300, 400, 600, 700, 900A, 900B, and 1400 is not intended to be limiting. However, those variations and modifications may not depart from the protection of the present disclosure.

FIG. 16 is a schematic diagram illustrating an exemplary imaging device 1600 according to some embodiments of the present disclosure.

As illustrated in FIG. 16, an imaging device 1600 may include a processor 1610 and a display 1620. The processor 1610 may be configured to implement the functions and/or methods disclosed in the present disclosure. The display 1620 may be configured to present data processed or generated by the functions and/or methods disclosed in the present disclosure. For example, the display 1620 may be configured to present the presentation interface 1100 and/or the presentation interface 1500.

Merely for illustration, only one processor is described in FIG. 16. However, it should be noted that the computing device 1600 in the present disclosure may also include multiple processors. Thus operations and/or method steps that are performed by one processor as described in the present disclosure may also be jointly or separately performed by the multiple processors. For example, if the processor of the computing device 1600 in the present disclosure executes both operation A and operation B, it should be understood that operation A and operation B may also be performed by two or more different processors jointly or separately (e.g., a first processor executes operation A and a second processor executes operation B, or the first and second processors jointly execute operations A and B).

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended for those skilled in the art, though not expressly stated herein. These alterations, improvements, and modifications are intended to be suggested by this disclosure, and are within the scope of the exemplary embodiments of this disclosure.

Moreover, certain terminology has been used to describe embodiments of the present disclosure. For example, the terms "one embodiment," "an embodiment," and/or "some embodiments" mean that a particular feature, structure, or characteristic described in connection with the embodiment is included in at least one embodiment of the present disclosure. Therefore, it is emphasized and should be appreciated that two or more references to "an embodiment" or "one embodiment" or "an alternative embodiment" in various portions of this disclosure are not necessarily all referring to the same embodiment. Furthermore, the particular features, structures, or characteristics may be combined as suitable in one or more embodiments of the present disclosure.

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the scope of the disclosed embodiments. For example, although the implementation of various components described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various inventive embodiments. This method of disclosure, however, is not to be interpreted as reflecting an intention that the claimed subject matter requires more features than are expressly recited in each claim. Rather, inventive embodiments lie in less than all features of a single foregoing disclosed embodiment.

In some embodiments, the numbers expressing quantities or properties used to describe and claim certain embodiments of the application are to be understood as being modified in some instances by the term "about," "approximate," or "substantially." For example, "about," "approximate," or "substantially" may indicate $\pm 20\%$ variation of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable.

In closing, it is to be understood that the embodiments of the application disclosed herein are illustrative of the principles of the embodiments of the application. Other modifications that may be employed may be within the scope of the application. Thus, by way of example, but not of limitation, alternative configurations of the embodiments of the application may be utilized in accordance with the teachings herein. Accordingly, embodiments of the present application are not limited to that precisely as shown and described.

## Claims

1. A method for image processing, implemented on a computing device (1600) having at least one processor (1610) and at least one storage device (150), comprising:

   obtaining multiple black blood images of a first region of a target subject; and
   generating a first segment result by inputting the black blood images into multiple input channels of a blood vessel segmentation model, wherein

the first segment result includes blood vessel segments of first blood vessels in the first region,
the blood vessel segmentation model is a trained machine learning model, and
each of the black blood images is randomly designated as an input image of one of the input channels.

2. The method of claim 1, wherein to generate the first segment result, the blood vessel segmentation model is configured to generate a concatenation image of the input images of the multiple input channels, and generate the first segment result by processing the concatenation image.

3. The method of claim 1 or claim 2, wherein the blood vessel segmentation model is generated according to a training process including:

obtaining a plurality of training samples, each of the plurality of training samples including multiple sample black blood images of a sample region and gold standard blood vessel segments corresponding to the sample region; and
generating the blood vessel segmentation model by training an initial model using the plurality of training samples, wherein the initial model includes multiple input channels, and each of the sample black blood images is randomly designated as an input image of one of the input channels during the training process.

4. The method of claim 3, wherein the training samples are generated based on sample first black blood images and sample second black blood images, and the input channels of the blood vessel segmentation model include a first input channel and a second input channel, wherein

a first weight value of the first input channel is determined based on a proportion of a first count to a total count, the first count being a count of the sample first black blood images, the total count being a sum of the first count and a second count of the sample second black blood images; and
a second weight value of the second input channel is determined based on a proportion of the second count to the total count.

5. The method of claim 4, wherein the plurality of training samples include first training samples and second training samples,

each first training sample includes one of the sample first black blood images and a corresponding copy image, and
each second training sample includes one of the sample first black blood images and one of the sample second black blood images that correspond to the sample region.

6. The method of any one of claims 1-5, wherein, the multiple black blood images include a first black blood image and a second black blood image, the second black blood image being collected using a different Magnetic Resonance Imaging (MRI) sequence from the first black blood image.

7. The method of any one of claims 1-6, wherein the multiple black blood images include a first black blood image and a copy image of the first black blood image.

8. The method of any one of claims 1-7, wherein the generating a first segment result by inputting the black blood images into multiple input channels of a blood vessel segmentation model comprises:

obtaining a bright blood image of the first region of the target subject; and
generating the first segment result by inputting the black blood images and the bright blood image into the multiple input channels of the blood vessel segmentation model, wherein each of the black blood images and the bright blood image is randomly designated as an input image of one of the input channels.

9. The method of any one of claims 1-8, further comprising determining a centerline of the first blood vessels in the first region based on the first segment result by:

obtaining a bright blood image of the first region of the target subject;
obtaining a second segment result corresponding to the bright blood image by performing segment operation on the bright blood image, the second segment result including second blood vessel segments of the first blood vessels in the first region;

obtaining a target segment result by fusing the first segment result and the second segment result; and determining the centerline of the first blood vessels based on the target segment result.

10. The method of claim 9, wherein the determining the centerline of the first blood vessels based on the target segment result includes:

obtaining a first centerline of the first blood vessels by performing a centerline extraction operation on the target segment result;
obtaining a second centerline of the first blood vessels by performing an endpoint culling operation on the first centerline of the first blood vessels; and
determining the centerline of the first blood vessels by correcting, based on the target segment result, the second centerline of the first blood vessels.

11. The method of claim 10, wherein the determining the centerline of the first blood vessels by correcting, based on the target segment result, the second centerline of the first blood vessels includes:

determining, based on the second centerline of the first blood vessels, at least one bifurcation point in the target segment result; and
determining the centerline of the first blood vessels based on the at least one bifurcation point.

12. The method of claim 11, wherein the determining the centerline of the first blood vessels based on the at least one bifurcation point includes:

determining, based on the at least one bifurcation point, a third centerline of the first blood vessels;
obtaining vascular wall information of the first blood vessels based on at least one of the multiple black blood images; and
determining the centerline of the first blood vessels by correcting, based on the vascular wall information, the third centerline of the first blood vessels.

13. The method of claim 11, wherein the determining the centerline of the first blood vessels based on the at least one bifurcation point includes:

determining, based on the at least one bifurcation point, a third centerline of the first blood vessels;
obtaining the second segment result; and
determining the centerline of the first blood vessels by performing, based on the second segment result, an extension operation on the third centerline of the first blood vessels.

14. The method of any one of claims 1-13, wherein the multiple black blood images at least include a first image indicating the vascular wall in the first region of the target subject, further comprising:

obtaining a second image of the target subject, the second image indicating the vascular lumen in a second region of the target subject, the first region and the second region both including a target region of the target subject;
generating a registration result by registering the first image and the second image; and
generating a target image based on the registration result, the first image, and the second image.

15. The method of any one of claims 1-13, further comprising:

obtaining a third image of a third region of the target subject, both the first region and the third region including a target region;
determining first detection information of the target region by processing at least one of the multiple black blood images;
determining second detection information of the target region by processing the third image; and
determining at least one abnormal vascular region in the target region based on the first detection information and the second detection information.

**Patentansprüche**

1. Verfahren zur Bildverarbeitung, implementiert auf einer Rechenvorrichtung (1600) mit mindestens einem Prozessor (1610) und mindestens einem Speichermedium (150), umfassend:

   Erhalten mehrerer Schwarzblutbilder eines ersten Bereichs eines Zielobjekts; und
   Erzeugen eines ersten Segmentierungsergebnisses durch Eingeben der Schwarzblutbilder in mehrere Eingangskanäle eines Blutgefäßsegmentierungsmodells, wobei
   das erste Segmentergebnis Blutgefäßsegmente der ersten Blutgefäße in dem ersten Bereich beinhaltet,
   das Blutgefäßsegmentierungsmodell ein trainiertes maschinelles Lernmodell ist
   und
   jedes der Schwarzblutbilder zufällig als Eingangsbild für einen der Eingangskanäle festgelegt wird.

2. Verfahren nach Anspruch 1, wobei zum Erzeugen des ersten Segmentergebnisses das Blutgefäßsegmentierungsmodell so konfiguriert ist, dass es ein Verkettungsbild der Eingangsbilder der mehreren Eingangskanäle erzeugt und das erste Segmentergebnis durch Verarbeiten des Verkettungsbildes erzeugt.

3. Verfahren nach Anspruch 1 oder Anspruch 2, wobei das Blutgefäßsegmentierungsmodell gemäß einem Trainingsprozess erzeugt wird, der Folgendes beinhaltet:

   Erhalten einer Vielzahl von Trainingsproben, wobei jede der Vielzahl von Trainingsproben mehrere Probenschwarzblutbilder eines Probenbereichs und dem Probenbereich entsprechende Goldstandard-Blutgefäßsegmente beinhaltet; und
   Erzeugen des Blutgefäßsegmentierungsmodells, indem ein Ausgangsmodell anhand der Vielzahl von Trainingsproben trainiert wird, wobei das Ausgangsmodell mehrere Eingangskanäle beinhaltet, und jedes der Probenschwarzblutbilder während des Trainingsprozesses zufällig als Eingangsbild eines der Eingangskanäle festgelegt wird.

4. Verfahren nach Anspruch 3, wobei die Trainingsproben basierend auf ersten Probenschwarzblutbildern und zweiten Probenschwarzblutbildern erzeugt werden und die Eingangskanäle des Blutgefäßsegmentierungsmodells einen ersten und einen zweiten Eingangskanal beinhalten, wobei

   ein erster Gewichtungswert des ersten Eingangskanals basierend auf einem Verhältnis eines ersten Zählwerts zu einem Gesamtzählwert bestimmt wird, wobei der erste Zählwert die Anzahl der ersten Probenschwarzblutbilder ist und der Gesamtzählwert die Summe aus dem ersten Zählwert und einem zweiten Zählwert der zweiten Probenschwarzblutbilder ist; und
   ein zweiter Gewichtungswert des zweiten Eingangskanals basierend auf einem Anteil des zweiten Zählwerts am Gesamtzählwert bestimmt wird.

5. Verfahren nach Anspruch 4, wobei die Vielzahl von Trainingsproben erste Trainingsproben und zweite Trainingsproben beinhaltet,

   jede erste Trainingsprobe eines der ersten Probenschwarzblutbilder und ein entsprechendes Kopiebild beinhaltet und
   jede zweite Trainingsprobe eines der ersten Probenschwarzblutbilder und eines der zweiten Probenschwarzblutbilder, die dem Probenbereich entsprechen, beinhaltet.

6. Verfahren nach einem der Ansprüche 1-5, wobei die mehreren Schwarzblutbilder ein erstes Schwarzblutbild und ein zweites Schwarzblutbild beinhalten, wobei das zweite Schwarzblutbild mit einer anderen Magnetresonanztomographie-Sequenz (MRI) als das erste Schwarzblutbild aufgenommen wird.

7. Verfahren nach einem der Ansprüche 1-6, wobei die mehreren Schwarzblutbilder ein erstes Schwarzblutbild und ein Kopiebild des ersten Schwarzblutbildes beinhalten.

8. Verfahren nach einem der Ansprüche 1-7, wobei das Erzeugen eines ersten Segmentergebnisses durch Eingeben der Schwarzblutbilder in mehrere Eingangskanäle eines Blutgefäßsegmentierungsmodells Folgendes umfasst:

   Erhalten eines Bildes hellen Blutes des ersten Bereichs des Zielobjekts; und

Erzeugen des ersten Segmentergebnisses, indem die Schwarzblutbilder und das Bild hellen Blutes in die mehreren Eingangskanäle des Blutgefäßsegmentierungsmodells eingegeben werden, wobei jedes der Schwarzblutbilder und das Bild des hellen Blutes zufällig als Eingangsbild eines der Eingangskanäle festgelegt werden.

9. Verfahren nach einem der Ansprüche 1-8, ferner umfassend Bestimmen einer Mittellinie der ersten Blutgefäße in dem ersten Bereich basierend auf dem Ergebnis des ersten Segments durch:

Erhalten eines Bildes hellen Blutes des ersten Bereichs des Zielobjekts;
Erhalten eines zweiten Segmentergebnisses, das dem Bild hellen Blutes entspricht, durch Durchführen einer Segmentierungsoperation an dem Bild hellen Blutes, wobei das zweite Segmentergebnis zweite Blutgefäßsegmente der ersten Blutgefäße in dem ersten Bereich beinhaltet,
Erhalten eines Zielsegmentergebnisses durch Verschmelzen des ersten Segmentergebnisses und des zweiten Segmentergebnisses; und
Bestimmen der Mittellinie der ersten Blutgefäße basierend auf dem Zielsegmentergebnis.

10. Verfahren nach Anspruch 9, wobei das Bestimmen der Mittellinie der ersten Blutgefäße basierend auf dem Zielsegmentergebnis Folgendes beinhaltet:

Erhalten der ersten Mittellinie der ersten Blutgefäße durch Durchführen einer Mittellinienextraktionsoperation am Zielsegmentergebnis;
Erhalten einer zweiten Mittellinie der ersten Blutgefäße durch Durchführen einer Endpunkt-Culling-Operation an der ersten Mittellinie der ersten Blutgefäße; und
Bestimmen der Mittellinie der ersten Blutgefäße durch Korrigieren, basierend auf dem Zielsegmentergebnis, der zweiten Mittellinie der ersten Blutgefäße.

11. Verfahren nach Anspruch 10, wobei das Bestimmen der Mittellinie der ersten Blutgefäße durch Korrigieren der zweiten Mittellinie der ersten Blutgefäße basierend auf dem Zielsegmentergebnis Folgendes beinhaltet:

Bestimmen mindestens eines Verzweigungspunktes im Zielsegmentergebnis basierend auf der zweiten Mittellinie der ersten Blutgefäße; und
Bestimmen der Mittellinie der ersten Blutgefäße basierend auf dem mindestens einen Verzweigungspunkt.

12. Verfahren nach Anspruch 11, wobei das Bestimmen der Mittellinie der ersten Blutgefäße basierend auf dem mindestens einen Verzweigungspunkt Folgendes beinhaltet:

Bestimmen einer dritten Mittellinie der ersten Blutgefäße basierend auf dem mindestens einen Verzweigungspunkt;
Erhalten von Informationen über die Gefäßwand der ersten Blutgefäße basierend auf mindestens einem der mehreren Schwarzblutbilder; und
Bestimmen der Mittellinie der ersten Blutgefäße durch Korrigieren der dritten Mittellinie der ersten Blutgefäße basierend auf Informationen über die Gefäßwand.

13. Verfahren nach Anspruch 11, wobei das Bestimmen der Mittellinie der ersten Blutgefäße basierend auf dem mindestens einen Verzweigungspunkt Folgendes beinhaltet:

Bestimmen einer dritten Mittellinie der ersten Blutgefäße basierend auf dem mindestens einen Verzweigungspunkt;
Erhalten des zweiten Segmentergebnisses; und
Bestimmen der Mittellinie der ersten Blutgefäße durch Durchführen, basierend auf dem zweiten Segmentergebnis, einer Verlängerungsoperation an der dritten Mittellinie der ersten Blutgefäße.

14. Verfahren nach einem der Ansprüche 1-13, wobei die mehreren Schwarzblutbilder mindestens ein erstes Bild beinhalten, das die Gefäßwand im ersten Bereich des Zielobjekts darstellt, ferner umfassend:

Erhalten eines zweiten Bildes des Zielobjekts, wobei das zweite Bild das Gefäßlumen in einem zweiten Bereich des Zielobjekts zeigt, wobei sowohl der erste Bereich als auch der zweite Bereich einen Zielbereich des Zielobjekts beinhalten;

Erzeugen eines Registrierungsergebnisses durch Registrieren des ersten Bildes und des zweiten Bildes; und Erzeugen eines Zielbildes basierend auf dem Registrierungsergebnis, dem ersten Bild und dem zweiten Bild.

15. Verfahren nach einem der Ansprüche 1-13, ferner umfassend:

Erhalten eines dritten Bildes eines dritten Bereichs des Zielobjekts, wobei sowohl der erste Bereich als auch der dritte Bereich einen Zielbereich beinhalten;
Bestimmen erster Detektionsinformationen des Zielbereichs durch Verarbeiten mindestens eines der mehreren Schwarzblutbilder;
Bestimmen zweiter Detektionsinformationen des Zielbereichs durch Verarbeiten des dritten Bildes; und
Bestimmen mindestens eines abnormalen Gefäßbereichs in dem Zielbereich basierend auf der ersten Detektionsinformationen und der zweiten Detektionsinformationen.

**Revendications**

1. Procédé de traitement d'image, mis en œuvre sur un dispositif informatique (1600) présentant au moins un processeur (1610) et au moins un dispositif de stockage (150), comprenant :

l'obtention de plusieurs images de sang noir d'une première région d'un sujet cible ; et
la génération d'un premier résultat de segment en introduisant les images de sang noir dans de multiples canaux d'entrée d'un modèle de segmentation de vaisseaux sanguins, dans lequel
le premier résultat de segment inclut des segments de vaisseaux sanguins de premiers vaisseaux sanguins dans la première région,
le modèle de segmentation de vaisseaux sanguins est un modèle d'apprentissage automatique entraîné, et
chacune des images de sang noir est désignée aléatoirement comme image d'entrée de l'un des canaux d'entrée.

2. Procédé selon la revendication 1, dans lequel, pour générer le premier résultat de segment, le modèle de segmentation de vaisseaux sanguins est configuré pour générer une image de concaténation des images d'entrée des multiples canaux d'entrée, et générer le premier résultat de segment en traitant l'image de concaténation.

3. Procédé selon la revendication 1 ou la revendication 2, dans lequel le modèle de segmentation de vaisseaux sanguins est généré selon un processus d'apprentissage incluant :

l'obtention d'une pluralité d'échantillons d'entraînement, chacun de la pluralité d'échantillons d'entraînement incluant plusieurs images de sang noir d'échantillon d'une région d'échantillon et des segments de vaisseaux sanguins de référence correspondant à la région d'échantillon ; et
la génération du modèle de segmentation de vaisseaux sanguins par l'entraînement d'un modèle initial à l'aide de la pluralité d'échantillons d'entraînement, dans lequel le modèle initial inclut plusieurs canaux d'entrée, et chacune des images de sang noir d'échantillon est désignée aléatoirement comme image d'entrée de l'un des canaux d'entrée au cours du processus d'entraînement.

4. Procédé selon la revendication 3, dans lequel les échantillons d'entraînement sont générés sur la base de premières images de sang noir d'échantillon et de secondes images de sang noir d'échantillon, et les canaux d'entrée du modèle de segmentation de vaisseaux sanguins incluent un premier canal d'entrée et un second canal d'entrée, dans lequel

une première valeur de poids du premier canal d'entrée est déterminée sur la base d'une proportion d'un premier comptage par rapport à un comptage total, le premier comptage étant un comptage des premières images de sang noir d'échantillon, le comptage total étant une somme du premier comptage et d'un second comptage des secondes images de sang noir d'échantillon ;
une seconde valeur de poids du second canal d'entrée est déterminée sur la base d'une proportion du second comptage par rapport au comptage total.

5. Procédé selon la revendication 4, dans lequel la pluralité d'échantillons d'entraînement inclut des premiers échantillons d'entraînement et des seconds échantillons d'entraînement,

chaque premier échantillon d'entraînement inclut l'une des premières images de sang noir d'échantillon et une image de copie correspondante, et

chaque second échantillon d'entraînement inclut une des premières images de sang noir d'échantillon et une des secondes images de sang noir d'échantillon qui correspondent à la région d'échantillon.

6. Procédé selon l'une quelconque des revendications 1-5, dans lequel les multiples images de sang noir incluent une première image de sang noir et une deuxième image de sang noir, la deuxième image de sang noir étant collectée à l'aide d'une séquence d'imagerie par résonance magnétique (IRM) différente de celle de la première image de sang noir.

7. Procédé selon l'une quelconque des revendications 1-6, dans lequel les multiples images de sang noir incluent une première image de sang noir et une image de copie de la première image de sang noir.

8. Procédé selon l'une quelconque des revendications 1-7, dans lequel la génération d'un premier résultat de segment par l'entrée des images de sang noir dans de multiples canaux d'entrée d'un modèle de segmentation de vaisseaux sanguins comprend :

l'obtention d'une image de sang clair de la première région du sujet cible ; et

la génération du premier résultat de segment par l'entrée des images de sang noir et de l'image de sang clair dans les multiples canaux d'entrée du modèle de segmentation de vaisseaux sanguins, dans lequel chacune des images de sang noir et de l'image de sang clair est désignée aléatoirement comme image d'entrée de l'un des canaux d'entrée.

9. Procédé selon l'une quelconque des revendications 1-8, comprenant en outre la détermination d'une ligne médiane des premiers vaisseaux sanguins dans la première région sur la base du premier résultat de segment par :

l'obtention d'une image de sang clair de la première région du sujet cible ;

l'obtention d'un second résultat de segment correspondant à l'image de sang clair par la réalisation d'une opération de segment sur l'image de sang clair, le second résultat de segment incluant des seconds segments de vaisseaux sanguins des premiers vaisseaux sanguins dans la première région ;

l'obtention d'un résultat de segment cible par la fusion du premier résultat de segment et du second résultat de segment ; et

la détermination de la ligne médiane des premiers vaisseaux sanguins sur la base du résultat de segment cible.

10. Procédé selon la revendication 9, dans lequel la détermination de la ligne médiane des premiers vaisseaux sanguins sur la base du résultat de segment cible inclut :

l'obtention d'une première ligne médiane des premiers vaisseaux sanguins par la réalisation d'une opération d'extraction de ligne médiane sur le résultat de segment cible ;

l'obtention d'une deuxième ligne médiane des premiers vaisseaux sanguins par la réalisation d'une opération d'élimination de points d'extrémité sur la première ligne médiane des premiers vaisseaux sanguins ; et

la détermination de la ligne médiane des premiers vaisseaux sanguins par la correction, sur la base du résultat de segment cible, de la deuxième ligne médiane des premiers vaisseaux sanguins.

11. Procédé selon la revendication 10, dans lequel la détermination de la ligne médiane des premiers vaisseaux sanguins par la correction, sur la base du résultat de segment cible, de la deuxième ligne médiane des premiers vaisseaux sanguins inclut :

la détermination, sur la base de la deuxième ligne médiane des premiers vaisseaux sanguins, d'au moins un point de bifurcation dans le résultat de segment cible ; et

la détermination de la ligne médiane des premiers vaisseaux sanguins sur la base de l'au moins un point de bifurcation.

12. Procédé selon la revendication 11, dans lequel la détermination de la ligne médiane des premiers vaisseaux sanguins sur la base de l'au moins un point de bifurcation inclut :

la détermination, sur la base de l'au moins un point de bifurcation, d'une troisième ligne médiane des premiers vaisseaux sanguins ;

l'obtention d'informations de paroi vasculaire des premiers vaisseaux sanguins sur la base d'au moins une des multiples images de sang noir ; et

la détermination de la ligne médiane des premiers vaisseaux sanguins par la correction, sur la base des informations de paroi vasculaire, de la troisième ligne médiane des premiers vaisseaux sanguins.

13. Procédé selon la revendication 11, dans lequel la détermination de la ligne médiane des premiers vaisseaux sanguins sur la base de l'au moins un point de bifurcation inclut :

la détermination, sur la base de l'au moins un point de bifurcation, d'une troisième ligne médiane des premiers vaisseaux sanguins ;

l'obtention du second résultat de segment ; et

la détermination de la ligne médiane des premiers vaisseaux sanguins par la réalisation, sur la base du second résultat de segment, d'une opération d'extension sur la troisième ligne médiane des premiers vaisseaux sanguins.

14. Procédé selon l'une quelconque des revendications 1-13, dans lequel les multiples images de sang noir incluent au moins une première image indiquant la paroi vasculaire dans la première région du sujet cible, comprenant en outre :

l'obtention d'une deuxième image du sujet cible, la deuxième image indiquant la lumière vasculaire dans une deuxième région du sujet cible, la première région et la deuxième région incluant toutes deux une région cible du sujet cible ;

la génération d'un résultat de recalage par le recalage de la première image et de la deuxième image ; et

la génération d'une image cible sur la base du résultat de recalage, de la première image et de la deuxième image.

15. Procédé selon l'une quelconque des revendications 1-13, comprenant en outre :

l'obtention d'une troisième image d'une troisième région du sujet cible, la première région et la troisième région incluant une région cible ;

la détermination de premières informations de détection de la région cible par le traitement d'au moins une des multiples images de sang noir ;

la détermination de secondes informations de détection de la région cible par le traitement de la troisième image ; et

la détermination d'au moins une région vasculaire anormale dans la région cible sur la base des premières informations de détection et des secondes informations de détection.

**100**

**FIG. 1**

<u>140</u>

Obtaining Module 210

Generation Module 220

Training Module 230

**FIG. 2**

300

**FIG. 3**

**400**

410

Black blood image 302 | Black blood image 304 | ... ...

Bright blood image 415

320

First input channel 422 | Second input channel 424 | Third input channel 426 | ... ...

430

Concatenation image

Bright blood image 450

330

First segment result

Second segment result 460

Target segment result 470

Centerline 440

**FIG. 4**

**500**

302  304  330

510  540

520  530

**FIG. 5**

**600**

Obtaining a plurality of training samples, each of the plurality of training samples including multiple sample black blood images of a sample region and gold standard blood vessel segments corresponding to the sample region — 602

Generating a blood vessel segmentation model by training an initial model using the plurality of training samples, wherein the initial model includes multiple input channels, and each of the sample black blood images is randomly designated as an input image of one of the input channels during a training process — 604

**FIG. 6**

700

330                  460

First segment result     Second segment result

470

Target segment result

710

First centerline

720

Second centerline

730

Bifurcation point

740             750

Third centerline         Vascular wall information

440

Centerline

**FIG. 7**

FIG. 8

**900A**

310

First image

340

Second image

345

Registration result

910

Local image

350

Target image

**FIG. 9A**

**900B**

310

First image

340

Second image

345

Registration result

920

First extraction result

930

Second extraction result

350

Target image

**FIG. 9B**

**350**

1010                    1020

**FIG. 10**

**1100**

1110                    1120

**FIG. 11**

350

1210          1220          1230

**FIG. 12**

1300

1310                    1330                    1320

**FIG. 13**

**1400**

FIG. 14

1500

1510                                          1520

**FIG. 15A**

1500

1510              1530                    1520

**FIG. 15B**

1600

| Processor | 1610 |

| Displayer | 1620 |

**FIG. 16**

**REFERENCES CITED IN THE DESCRIPTION**

**Patent documents cited in the description**

- CN 202311828189
- CN 202311813012
- CN 202311826362
- US 2023237648 A1
- US 2023206444 A1

**Non-patent literature cited in the description**

- **RESHA DWIKA HEFNI AL-FAHSI et al.** MSGNet: Modified MobileNet-ShuffleNet-GhostNet Network for Lightweight Retinal Vessel Segmentation. *2023 10th International Conference on Information Technology, Computer, and Electrical Engineering (ICITACEE)*, 31 August 2023